# EUROPEAN PATENT SPECIFICATION

(11) **EP 3 492 031 B1**
(45) Date of publication and mention of the grant of the patent: **02.03.2022**
(21) Application number: 19151981.8
(22) Date of filing: 18.04.2013
(51) Int. Cl.: A61B 17/34, A61B 17/02

(54) **NATURAL ORIFICE SURGERY SYSTEM**
CHIRURGISCHES SYSTEM FÜR NATÜRLICHE ÖFFNUNG
SYSTÈME DE CHIRURGIE D'ORIFICE NATUREL

(30) Priority: 20.04.2012 US 201261636492 P
(43) Date of publication of application: 05.06.2019
(62) Divisional of application: 13721833.5
(73) Proprietor: Applied Medical Resources Corporation, Rancho Santa Margarita, CA 92688 (US)
(72) Inventor: DANG, Kevin K, Rancho Santa Margarita, CA California 92688 (US); ALBRECHT, Jeremy J, Rancho Santa Margarita, CA California 92688 (US); BROWN, Blaze, Rancho Santa Margarita, CA California 92688 (US); HOKE, Adam, Rancho Santa Margarita, CA California 92688 (US); SAIDUDDIN, Adeeb, Rancho Santa Margarita, CA California 92688 (US); JOHNSON, Gary, Rancho Santa Margarita, CA California 92688 (US); FILEK, Jacob J, Rancho Santa Margarita, CA California 92688 (US)
(74) Representative: Dolleymores

(56) References cited:
- EP-A1- 1 852 053
- WO-A2-2007/109700
- US-A- 4 117 847
- US-A1- 2007 213 675
- US-A1- 2010 261 951
- US-A1- 2012 095 297

## Description

### BACKGROUND

### Technical Field

This application is generally directed to surgical devices, and more particularly, to a retractor adapted for use with a cap, that is useful in natural orifice single-port surgical procedures.

### Description of the Related Art

Access devices are commonly used in surgery to facilitate the introduction of various surgical instruments into natural biological vessels, conduits, orifices, cavities, and other interior regions of the body. These access devices include, for example, devices that facilitate the introduction of a needle into a vessel, and trocars that facilitate the introduction of laparoscopic instruments into the abdomen of the body.

Some of these access devices are introduced into regions that include a fluid or gas under pressure. In the case of a needle access device, the pressure may be from a liquid, such as blood. In the case of a trocar, the pressure may be from a gas, such as an insufflation gas. In either case, it is desirable to provide for the introduction of the surgical instrument into the cavity without permitting the escape of the pressurized fluid or gas.

In the case of trocars, a cannula at the distal end of the trocar is typically connected to a seal housing at the proximal end of the trocar. Together the cannula and housing form a working channel through which various instruments can be inserted to access the cavity. Seal mechanisms are commonly disposed in the housing and include a septum valve that seals the working channel when an instrument is in place, and a zero closure valve that seals the working channel when the instrument is removed. Examples of such devices can be found in US patent application, publication number US 2012/0095297 A1, European patent application, publication number EP 1852053 A1, and International patent application number WO 2007/109700 A2.

Current surgical access ports allow for single instrument access through each port, or allow for multiple instrument access through a rigid cannula. Some devices, such as transanal endoscopic microsurgery (TEMS) units require that the device be attached to the surgical table to support the weight of the device, as well as to locate the position of the device respective to the patient. These devices do not provide flexibility to the surgeon in selecting instrument size, and they restrict instrument movement with their rigid cannulas. Additionally, surgeons are performing laparoscopic surgical procedures through a single or a limited number of access ports. The procedures may be performed through a single two (2) centimeter incision at the umbilicus, or in certain cases, trans-vaginally or trans-anally. What is needed is a system that meets the needs of these new procedures, facilitating more flexible movement of laparoscopic instruments through a single or limited number of ports while preventing the escape of pressured fluids or gasses and permitting large specimen removal.

### SUMMARY OF THE INVENTION

According to a first aspect of the present invention there is provided a surgical access port system, as claimed in claim 1, adapted for performing laparoscopic surgical procedures at a natural orifice comprising a retractor comprising: an outer ring, wherein the outer ring is configured to be disposed proximate the natural orifice of the patient and substantially surround the orifice; a tubular body, wherein the tubular body defines a generally cylindrical passage large enough to accommodate at least one laparoscopic instrument there through; a funnel segment extending between and coupling the outer ring and the tubular body, wherein the funnel segment provides a diametric reduction between the relatively large diameter of the outer ring and the relatively smaller diameter of the tubular body, which is sized to fit within a natural orifice with minimal distention of the orifice; and an inflatable member disposed on a distal edge of the tubular body, characterized in further comprising; and an inflation port disposed within the funnel segment; and a channel disposed within the wall of the tubular body, wherein a proximal opening of the channel communicates with the inflation port and a distal opening of the channel communicates with the inflatable member, and wherein the inflatable member has a pre-molded shape suitable for use with particular natural orifices.

According to a second aspect of the present invention there is provided a method for making the surgical access port system, as claimed in claim 5, wherein the method comprises the steps of: heat shrinking the polyolefin tubing around an exterior surface of the distal end of the tubular body, heating the distal end of the tubular body and placing the distal end of the tubular body inside a mold having a desired shape of the inflatable member when inflated, and injecting a gas into the inflatable member through the inflation port and channel with the distal end of the tubular body still inside the mold to thereby produce the desired pre-molded shape of the inflatable member. Further embodiments of the invention are defined by the dependent claims.

These and other features and advantages of the invention will become more apparent with a discussion of examples in reference to the associated drawings.

### BRIEF DESCRIPTION OF THE DRAWINGS

FIG. **1** is a side view of a patient in surgery illustrating an example of an access device positioned on the abdomen and in use.
FIG. **2** is a cross-sectional side view illustrating an example of the access device, with the wound retractor retracting the vagina of a patient, and a gel cap sealing the opening of the wound retractor.
FIG. **3** is a front view illustrating an example of the access device deployed and in use at the mouth of the patient.
FIG. **4** is a top view illustrating a patient in the prone position with an example of the access device deployed and in use at the anus of the patient.
FIG. **5** is a perspective view of an example of an access device comprising a cap and a retractor.
FIG. **6A** is a side view of an example of a natural orifice retractor. FIG. **6B** is a top view of the natural orifice retractor of FIG. **6A****.** FIG. **6C** is a partial cut away of the natural orifice retractor of FIG. **6A****.**
FIG. **6D** is a side view of another example of a natural orifice retractor. FIG. **6E** is a top view of the natural orifice retractor of FIG. **6D****.** FIG. **6F** is a perspective view of the natural orifice retractor of FIG. **6A****.**
FIG. **6G** is a perspective view of an obturator adapted to facilitate introduction of a natural orifice retractor into a body orifice such as an anus. FIG. **6H** is a side view of the obturator of FIG. **6G****.**
FIG. **6I** is a perspective view of an obturator having a straight shaft piece, adapted to facilitate introduction of a natural orifice retractor into a body orifice such as an anus. FIG. **6J** is a perspective view of a retractor disposed on the obturator of FIG. **6I****.**
FIG. **7A** is a partial side cross section of the natural orifice retractor of FIG. **6A** with a gel cap coupled therewith to form one example of natural orifice access device.
FIG. **7B** is a side cross section of the natural orifice retractor of FIG. **6D****.**
FIG. **7C** is a perspective view of a natural orifice retractor formed from sections and having cut-out portions or windows in the tubular body of the retractor. FIG. **7D** is a cutaway view of the retractor of FIG. **7C** showing the slidable engagement of the sections. FIG. **7E** is a cutaway view of the retractor of FIG. **7C** showing the snap-lock mechanism securing the sections together.
FIG. **7F** is a perspective view and a side view of an alternative example of a retractor having cut-out portions or windows in the tubular body of the retractor.
FIG. **7G** is a perspective view of an example of a retractor in accordance with the present invention, having an inflatable member.
FIG. **7H** shows a close-up view of the inflatable member of FIG. **7G****.** FIG. **7I** is a top-down perspective view of the retractor of FIG. **7G** showing the check valve port of inflating the inflatable member. FIG. **7J** is a cutaway side view showing the check valve and channel disposed in the tubular body of the retractor. FIG. **7K** is a side view of a retractor showing the channel disposed between the check valve and the inflatable member.
FIG. **7L** is a perspective view of an obturator, modified with an indent to provide clearance for the inflation port shown in FIG. **7J** and **7K** and adapted to facilitate introduction of a natural orifice retractor into a body orifice such as an anus.
FIG. **7M** is a perspective view and a side view of a perforated natural orifice retractor.
FIG. **8A** is a side view of the natural orifice access device of FIG. **7A****.** FIG. **8B** is a top view of the natural orifice access device illustrated in FIG. **7A****.** FIG. **8C** is a perspective view of the natural orifice access device illustrated in FIG. **7A****.**
FIG. **8D** is a perspective view of the natural orifice retractor of FIG. **6D** with a gel cap therewith to form one example of natural orifice access device.
FIG. **9A** is a perspective view of an example of a natural orifice access device including a cap having a plurality of trocars extending there through. FIG. **9B** is a perspective view of another example of a natural orifice access device including a cap having a plurality of trocars extending there through.
FIG. **9C** is an exploded view of an example of a trocar access device and optional obturator, which is a component of some examples of the access device system.
FIG. **10A** is a top perspective view of an example of a gel cap. FIG. **10B** is a bottom view of an example of a cap ring.
FIG. **11A** is a top view of an example of a gel cap comprising a plurality of access ports embedded in the gel pad. FIG. **11B** is a top perspective view of the gel cap illustrated in FIG. **11A****.** FIG. **11C** is a bottom perspective view of the gel cap illustrated in FIG**. 11A****.**
FIG. **11D** is a top perspective view of the gel cap illustrated in FIG. **11A** with instruments inserted through two of the access ports. FIG. **11E** is a bottom perspective view of the gel cap and instruments illustrated in FIG. **11D****.** FIG. **11F** is a side view of the gel cap and instruments illustrated in FIG. **11D****.**
FIG. **11G** is a top perspective view of an example of gel cap comprising a fixed camera or laparoscope port.
FIG. **12** is a cutaway perspective view of an example of an access device system comprising a gel cap that snap fits to a retractor.
FIG. **13** is an exploded view of an example of a trocar.
FIGS. **14A** and **14B** are side views of an example of a trocar comprising a fixation cannula in an insertion configuration and a fixation configuration, respectively.
FIG. **15** is a side view of another example of a trocar comprising a fixation cannula.
FIG. **16A** is a side view of another example of a trocar comprising a fixation cannula. FIG. **16B** is a perspective view of an example of a bolster suitable for use with the trocar illustrated in FIG. **16A****.**
FIG. **17A** is a side view of another example of a trocar comprising a fixation cannula. FIG. **17B** is a perspective view of an example of a bolster suitable for use with the trocar illustrated in FIG. **17A****.**

Similar components have similar reference numbers throughout.

### DETAILED DESCRIPTION OF CERTAIN EXAMPLES

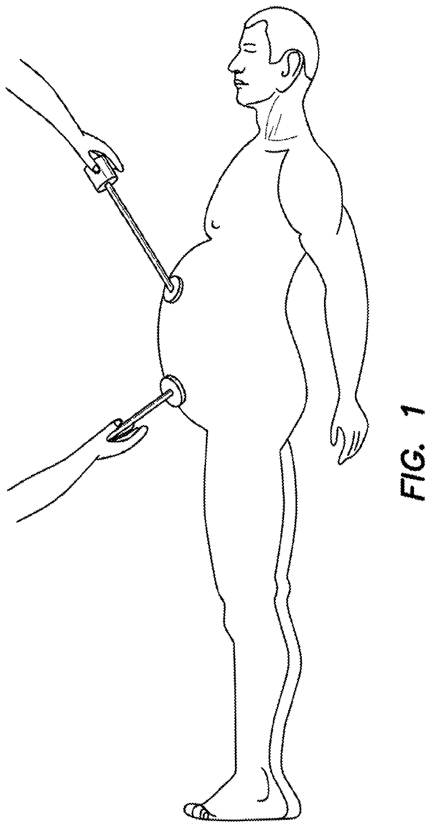

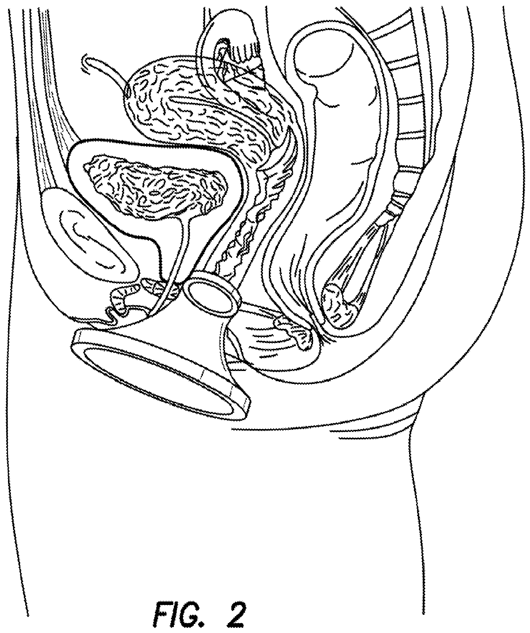

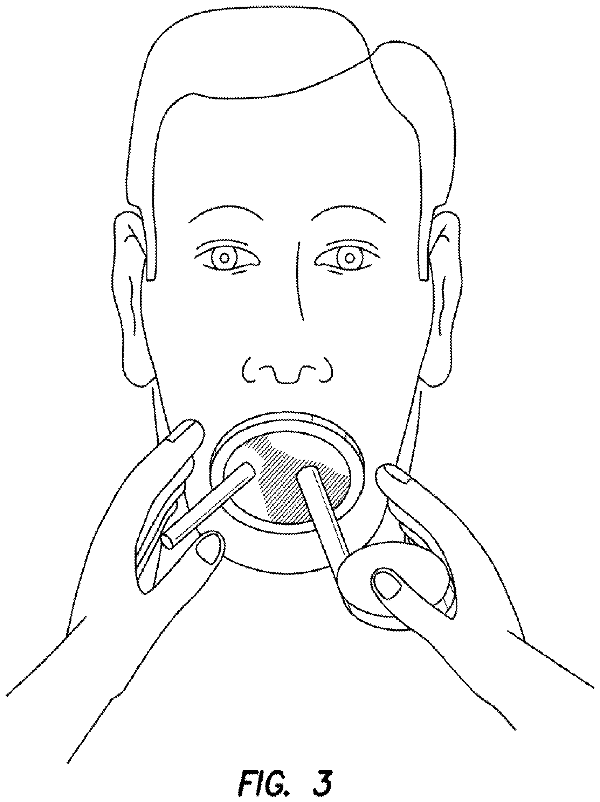

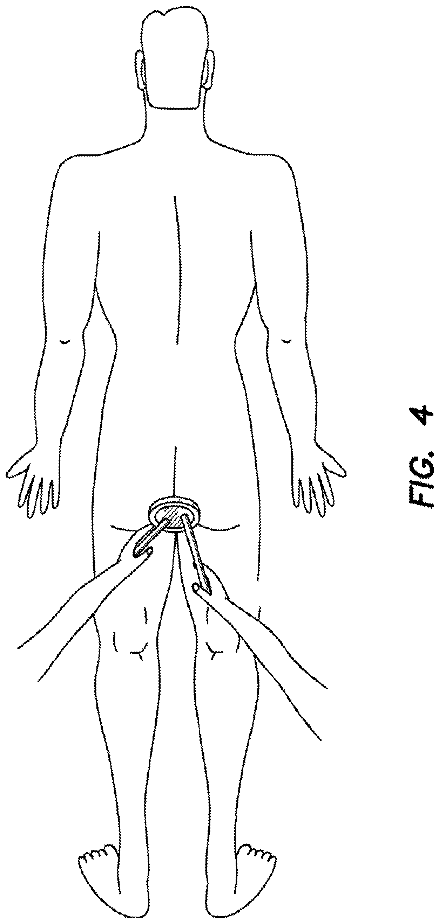

Examples of a surgical instrument access device system are useful, for example, for single incision, single port, and/or limited port laparoscopic surgical procedures, for example, abdominal (FIG. **1****),** transvaginal (FIG. **2****),** transoral (FIG. **3****),** and transanal (FIG. **4****)** procedures.

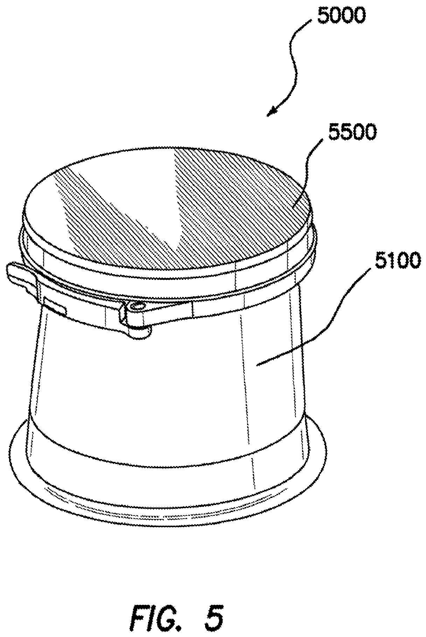

FIG. **5** illustrates a perspective view of an example of an access device system 5000 comprising a retractor **5100** and a cap **5500,** which is useful in single port and/or limited port procedures. The retractor or surgical wound retractor **5100** is placed and/or positioned into, across, and/or through a surgical incision and/or body orifice to enlarge, reshape, and/or isolate the incision or body orifice. The cap **5500** provides an artificial body wall through which instruments access the interior of a patient's body, for example, a body cavity. The components of the access device **5000** comprise any suitable biologically compatible materials.

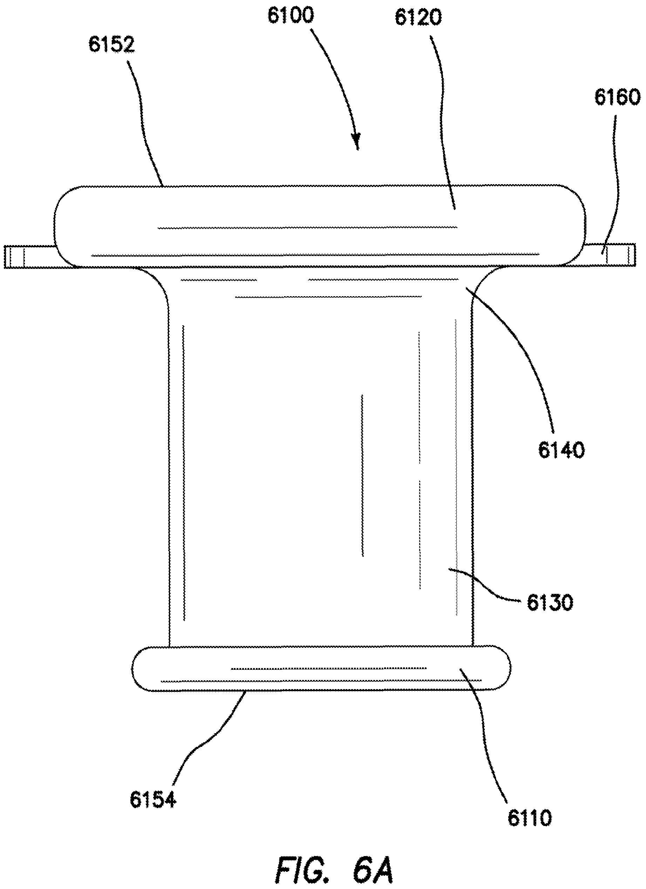

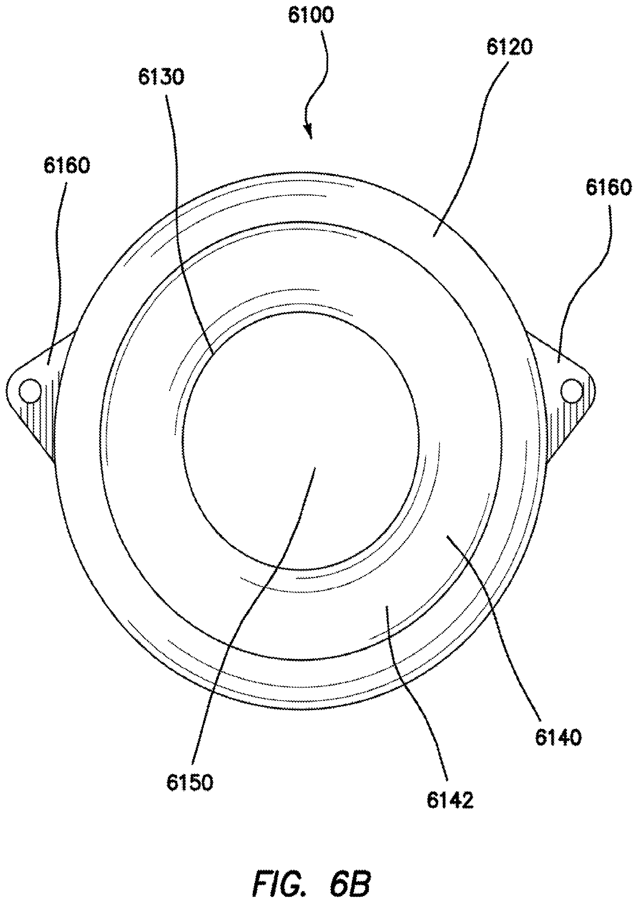

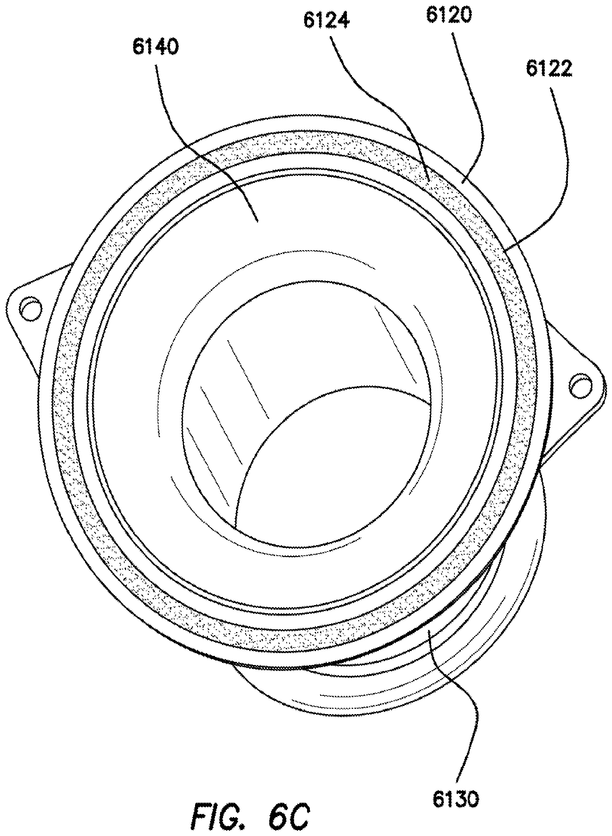

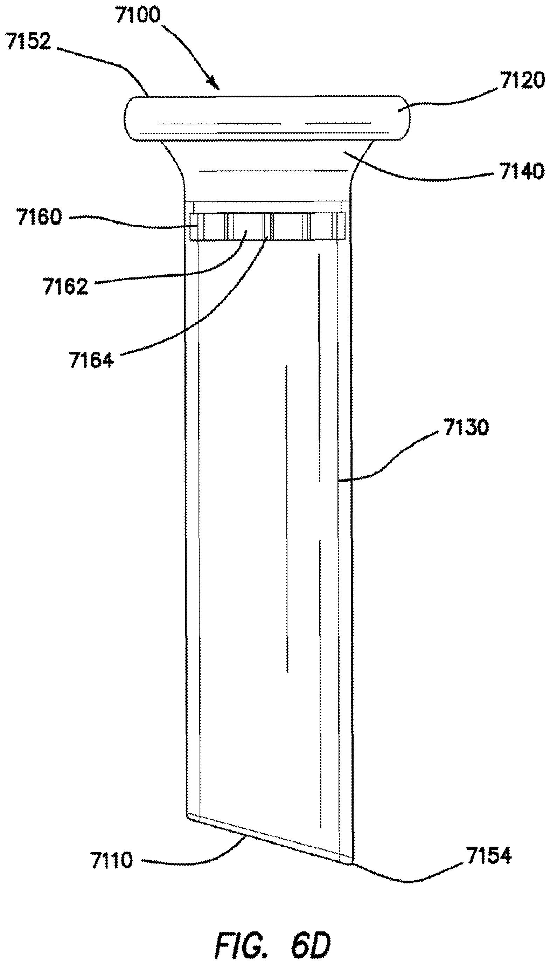

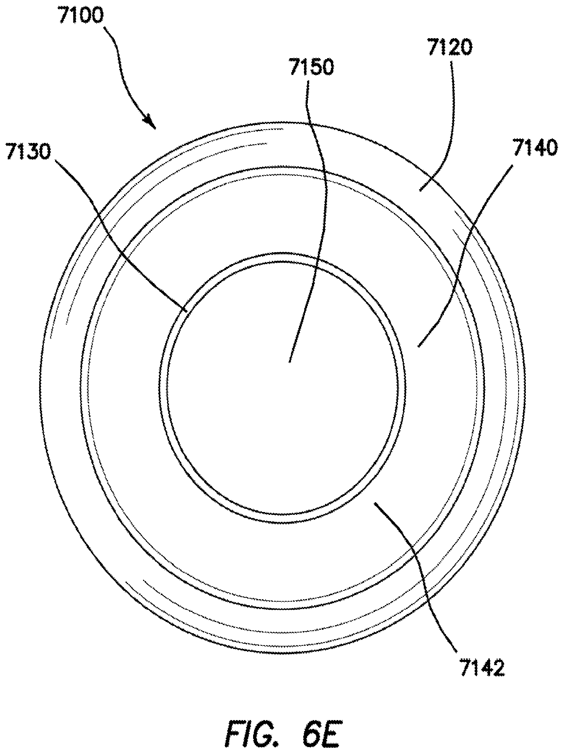

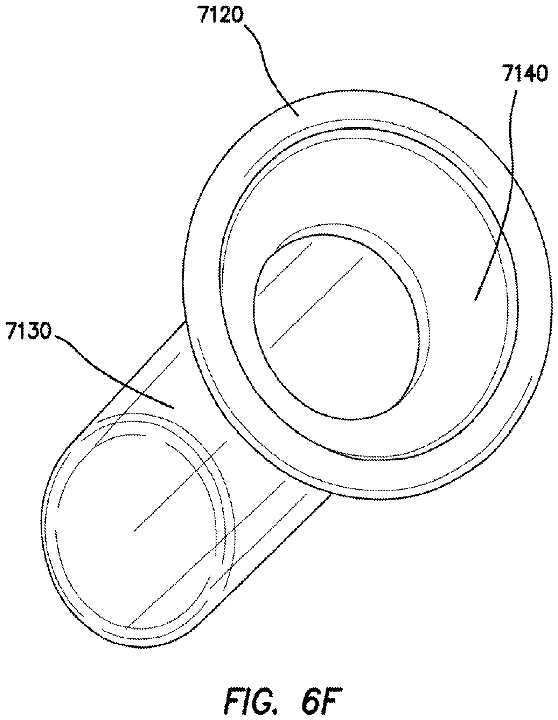

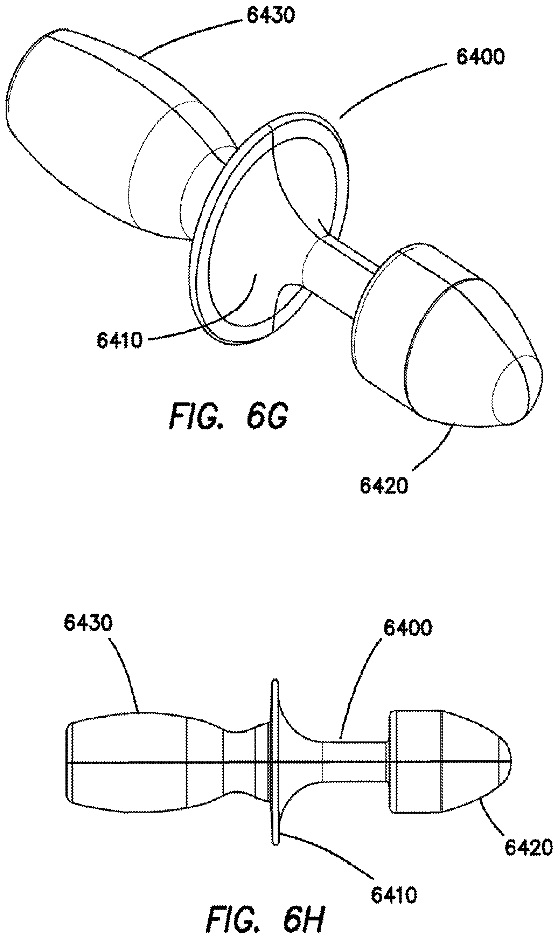

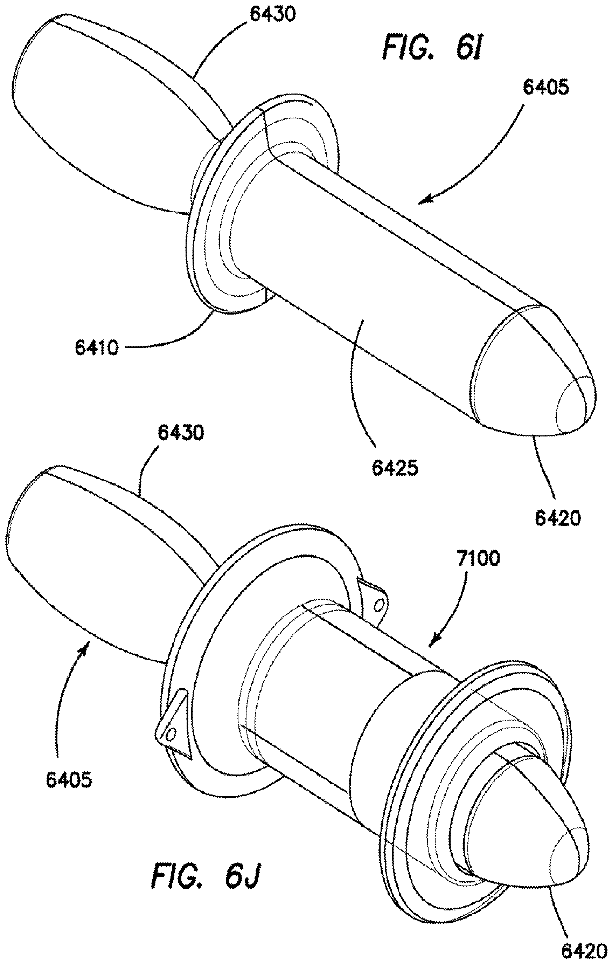

Two examples of natural orifice access ports or retractors **6100, 7100** sharing certain similarities are illustrated in FIGS. **6-9****.** One example of retractor **6100** is illustrated in FIGS. **6A-6C**, 7A, 8A-8C, and **9A.** Another example of retractor **7100** is illustrated in FIGS. **6D-6F**, 7B, 8D., and **9B**

The example of the natural orifice access port or retractor **6100** illustrated in a side view in FIG. **6A** can be adapted for use in a transanal surgical procedure. The retractor **6100** comprises an inner or distal ring **6110,** an outer or proximal ring **6120,** a tubular body **6130,** and a funnel segment **6140** extending between and coupling the inner ring **6110** and the outer ring **6120.** The tubular body **6130** comprises a relatively flexible material such as a KRATON^{®} material or a silicone rubber material, which is substantially cylindrical in the illustrated example. In other examples, the tubular body **6130** has another shape, for example, an oval cross section. Some examples of the tubular body **6130** comprise one or more coatings that provide additional functionality, for example, an anti-microbial coating.

Examples of the inner ring **6110** are sufficiently flexible and compliant to be compressed and/or deformed for insertion into a body orifice such as a patient's anus during a transanal surgical procedure. When subsequently released within an associated body cavity, the inner ring **6110** substantially returns to its original shape or footprint. In some examples, the inner ring **6110** assumes a substantially circular shape in a relaxed state, for example, when released within a body cavity. In other examples, the inner ring **6110** has another shape in the relaxed state, for example, an oval. The inner ring **6110** assumes a different shape when compressed for insertion through an incision or body orifice, for example, a substantially oval shape, a generally linear shape, a tear-drop shape, or another suitable shape. Those skilled in the art will recognize that in other examples, the inner ring **6110** in the relaxed state has a shape other than round, for example, oval, elliptical, or D-shaped. In other examples, the inner ring **6110** is substantially rigid, that is, non-compliant under the ordinary conditions under which it is used. In some examples, the inner ring extends outward from the surface of the tubular body, as shown, for example, in FIG. 6A, to thereby aid in retaining the retractor in the body cavity after it is deployed.

Examples of the inner ring **6110** can comprise a generally circular cross section. In other examples, the inner ring **6110** comprises another cross-sectional shape, for example, at least one of oval or elliptical, tear-drop shaped, and D-shaped. For example, in examples illustrated in FIGS. **6D-6F**, the inner ring **7110** can have a cross-sectional shape that is substantially flush with the tubular body **7130** of the retractor **7100** as further described herein. Those skilled in the art will understand that other cross sections are used in other examples. As further discussed herein with respect to the flexion region of the inner ring **6110,** some examples of the inner ring **6110** comprise at least one notch and/or weak spot, which facilitate folding or deforming the inner ring **6110,** thereby facilitating insertion and/or removal of the inner ring **6110.**

Returning to FIG. **6A****,** the outer ring **6120** is proximal the funnel section **6140.** In the illustrated example, the outer ring **6120** has a substantially circular footprint. As further discussed herein, the outer ring **6120** can be sized and configured to sealingly couple to a cap or other access device thereon. In some examples, one or more suture points **6160** can be disposed on the retractor **6110** adjacent the outer ring **6120.**

With reference to FIG. **6B****,** a top view of retractor **6100** is illustrated. In the illustrated example, outer ring **6120** has a generally circular profile. Additionally, in the illustrated example, two suture points **6160** are generally diametrically opposed relative to the generally circular profile of the outer ring **6120.** In other examples, the retractor can include more or fewer than two suture points disposed of various locations relative to the outer ring **6120.**

With continued reference to FIG. **6B****,** the tubular body **6130** has a generally circular profile defining a generally cylindrical passage **6150.** The generally cylindrical passage **6150** is desirably large enough to accommodate more than one laparoscopic instrument there through such that a single natural orifice access device can be used to provide access for multiple surgical instruments in a body cavity. Moreover, generally cylindrical passage **6150** is desirably large enough such that multiple surgical instruments positioned there through can be translated or pivoted relative to one another, allowing a surgeon to manipulate the instruments as desired during a surgical procedure. The generally cylindrical passage extends between a proximal end **6152** of the retractor **6100** adjacent the outer ring **6120** to a distal end **6154** of the retractor **6100** adjacent the inner ring **6110** (FIG. **6A****).**

With continued reference to FIG. **6B** the funnel segment **6140** provides a diametric reduction between the relatively large diameter of the outer ring **6120,** which is sized and configured to be removably coupled to an access device such as a cap, and the relatively smaller diameter of the passage **6150,** which is sized to fit within a natural orifice with minimal distention of the orifice. The funnel segment **6140** has an inner surface **6142** which can provide a bearing surface for an obturator used to advance to the retractor **6100** into a body cavity. The funnel segment **6140** can have a substantially linear taper between the relatively large diameter and the relatively smaller diameter such that the inner surface **6142** is a frusto-conical segment. In other examples, the funnel segment **6140** can have a curved profile between the relatively large diameter and the relatively smaller diameter.

In some examples, a natural orifice access system can include a retractor **6100** and an optional obturator **6400** (FIG. **6G-6H****).** The obturator can have a proximal bearing surface **6410** sized and configured to bear against the inner surface **6142** of the funnel segment **6140** and a distal dilation surface **6420** sized and configured to expand a natural orifice for passage of the retractor **6100.** Thus, during insertion of the retractor **6100** into a natural orifice, the dilation surface **6420** expands a pathway to a surgical site in a body cavity while the obturator bears on the inner surface **6142** of the funnel segment **6140** to advance the retractor **6100** into position in the surgical site. Furthermore, in some examples, the obturator can have a handle **6430** at a proximal end thereof adapted to facilitate selective twisting or rotation of the obturator about a longitudinal axis thereof during insertion.

It can be desirable that the outer ring **6120** is relatively stiff compared with the relatively flexible tubular body **6130** of the retractor **6100** so that the outer ring **6120** can sealingly engage an access device such as a cap. With reference to FIG. **6C****,** a perspective view of the retractor is illustrated with a partial cutaway of the outer ring **6120.** In the illustrated example, the outer ring **6120** includes an annular groove **6122** formed therein in which a reinforcing member **6124** is disposed. The reinforcing member **6124** can comprise a metallic member such as a wire formed into a ring shape. For example, the reinforcing member **6124** can comprise a stainless steel ring positioned within the groove **6122** during manufacture of the retractor **6100** or the reinforcing member **6124** can comprise an injectable nonmetallic member. For example a glass filled polymer or polycarbonate material can be injected into the groove **6122** during manufacture of the retractor **6100.**

While the illustrated examples of retractor **6100** include a reinforcing member to enhance the rigidity of the outer ring **6120** the retractor **6100** can be formed in a multiple-shot molding process. For example an inner segment of the retractor defined by the tubular body **6130** and the inner ring **6110** can be formed in one molding operation from a flexible material, and an outer segment of the retractor **6100** defined by the funnel segment **6140** and the outer ring **6120** is formed in another molding operation from a relatively rigid material such as a polycarbonate material or other suitable material. One example of retractor **7100** formed in a multiple-shot molding process is illustrated in FIGS. **6D**-F, 7B, **8D,** and **9B.**

With continued reference to FIG. **6C****,** the illustrated example includes a continuous generally annular groove. In other examples, a plurality of noncontiguous recesses can each receive one of a plurality of reinforcing members. Moreover, in some examples, the outer ring can include two or more concentric generally annular grooves, which each receive a corresponding reinforcing member.

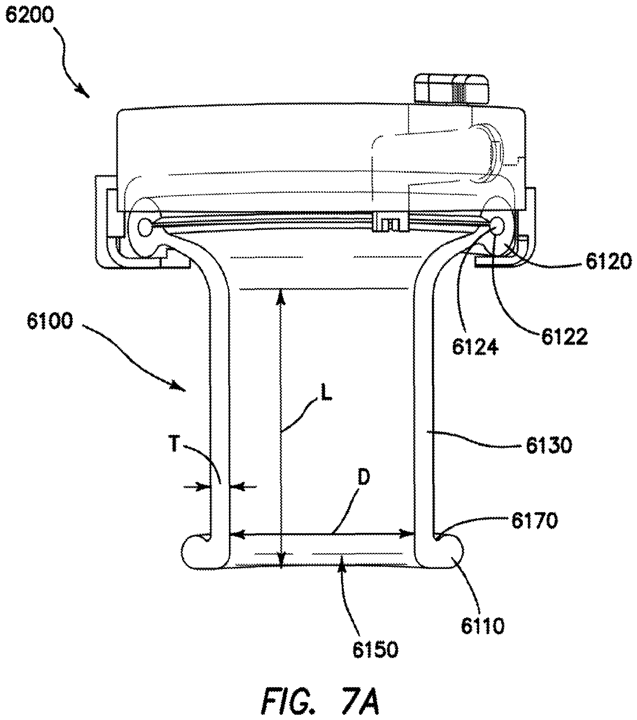

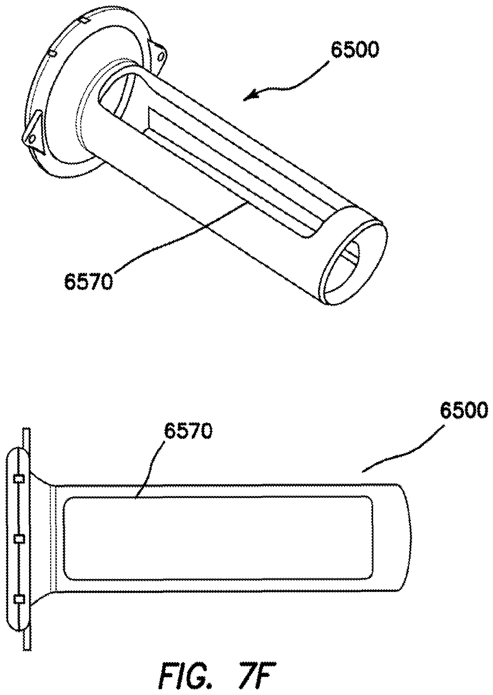

With reference to FIG. **7A****,** a cross-sectional view of a natural orifice access device including a retractor **6100** and a removable cap **6200** is shown. In the illustrated example, the tubular body **6130** is formed of a flexible material having a predetermined fixed length L, inner diameter D, and wall thickness T. The fixed length L, inner diameter D, and wall thickness T are selected to accommodate the anatomy of a natural orifice, such as the anal orifice of a majority of patients. It is contemplated that the retractor **6100** can be scaled to different sizes for patients of different ages. Furthermore, it is contemplated that the retractor can include a telescopic tubular body such that the tubular body can be selectively positioned at a variety of lengths depending on patient anatomy and the location of the surgical site within the body cavity. Desirably, the wall thickness T and material of the tubular body **6130** are selected such that the tubular body **6130** is resilient enough to maintain the passage **6150** there through when positioned in the natural orifice. Moreover, desirably, the inner diameter, D is sufficiently large to accommodate multiple surgical instruments. For example, where the retractor **6100** is adapted for use in a TEMS procedure, the inner diameter D and thickness T can be sized such that an outer diameter of the retractor can be between approximately 30 mm and 70 mm, desirably between approximately 35 mm and 50 mm, and preferably approximately 40 mm. Additionally, desirably, the fixed length L is sufficiently long such that the inner ring **6110** can be positioned at a surgical site within a body cavity and the outer ring **6120** can be positioned outside the natural orifice. In some examples, the fixed length L is of a length such that the device has an overall length between the proximal end **6152** and the distal end **6154** of between approximately 10 mm and approximately 100 mm, desirably between approximately 20 mm and 80 mm, more desirably between approximately 30 mm and 60 mm, and preferably, approximately 40 mm.

With continued reference to FIG. 7A the annular groove **6122** can be open to an inner surface of the outer ring **6120.** Thus, the retractor **6100** can be formed of a flexible material in a single molding operation with the annular groove **6122** having an opening, and the reinforcing member **6124** can be subsequently inserted into the upper groove **6122.**

With continued reference to FIG. **7A** the retractor **6100** can include a flexion region between the tubular body **6130** and the inner ring **6110,** such as an undercut **6170.** Advantageously, the flexion region can allow the inner ring **6110** to flex or rotate relative to the tubular body **6130** during insertion such that the inner ring **6110** presents a relatively small outer diameter in an insertion configuration and a relatively larger outer diameter in an undisturbed configuration.

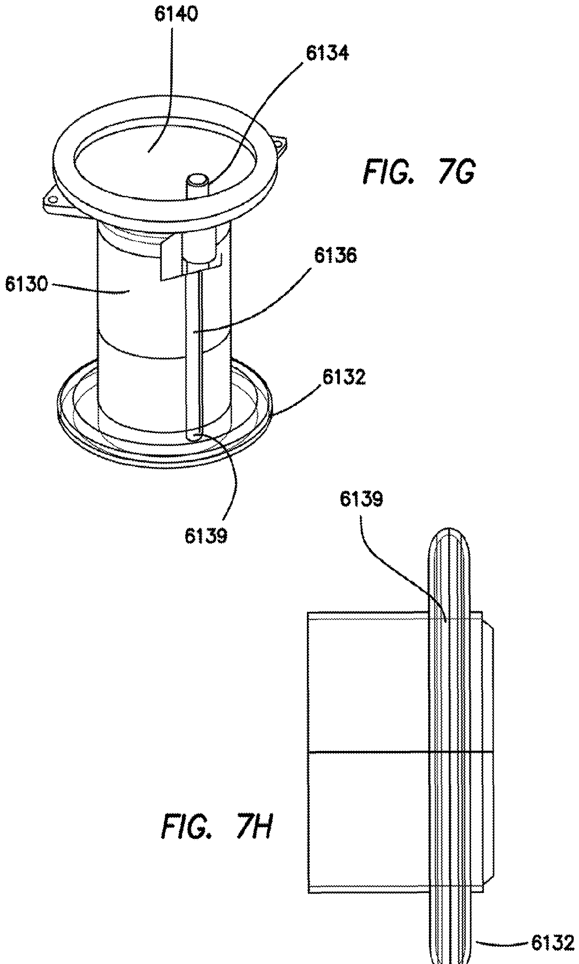

In an example in accordance with the present invention, and as shown in FIG. **7G****,** the inner ring can comprise an inflatable member **6132** such as an annular balloon coupled to a gas or fluid source that can be selectively inflated and deflated between a deflated, relatively small diameter state for insertion and removal, and an inflated, relatively high diameter state for retention in a body cavity. An inflation port **6134,** for example a check valve, affixed to the funnel portion **6140** of the retractor, is connected to the inflatable member **6132** through a channel **6136** within the wall of the tubular body **6130.** Fluid or gas introduced through the inflation port flows through the channel into the inflatable member to thereby inflate the member.

The channel **6136** runs through the tubular body, generally parallel to the longitudinal axis of the tubular body, with a proximal opening interacting with the inflation port **6134** and a distal opening **6139** into outer surface of the tubular body at the inflatable member. In one aspect, the inflation port **6134** may include a normally closed check valve having a spring-loaded plunger. In a further aspect, the check valve may include a Luer lock. It is contemplated that other inflation ports that are well known in the art may be used.

In this example, the tubular body **6130** is preferably comprised of a relatively rigid material, such as a polycarbonate. The tubular body has an inflatable member at the distal end that may be created by heat shrinking polyolefin tubing around the outside of the tubular body. The distal end of the body/tubing assembly is then heated for approximately 30 to 40 seconds, and then placed inside a mold and injected with air to give the inflatable member an annular balloon shape as seen in FIG. **7H****,** or any other desired shape, depending on the configuration of the mold. The inflatable member **6132** should have sufficient impermeability properties to substantially prevent inflation gas or fluid from permeating through a wall of the inflatable member.

In one example, the inflatable member **6132** may include a substantially toroid shape upon inflation. In another example, the inflatable member may include a disc shape upon inflation. In another example, the inflatable member **6132** may be a fluted balloon. Other shapes suitable for particular natural orifices will be appreciated by one skilled in the art.

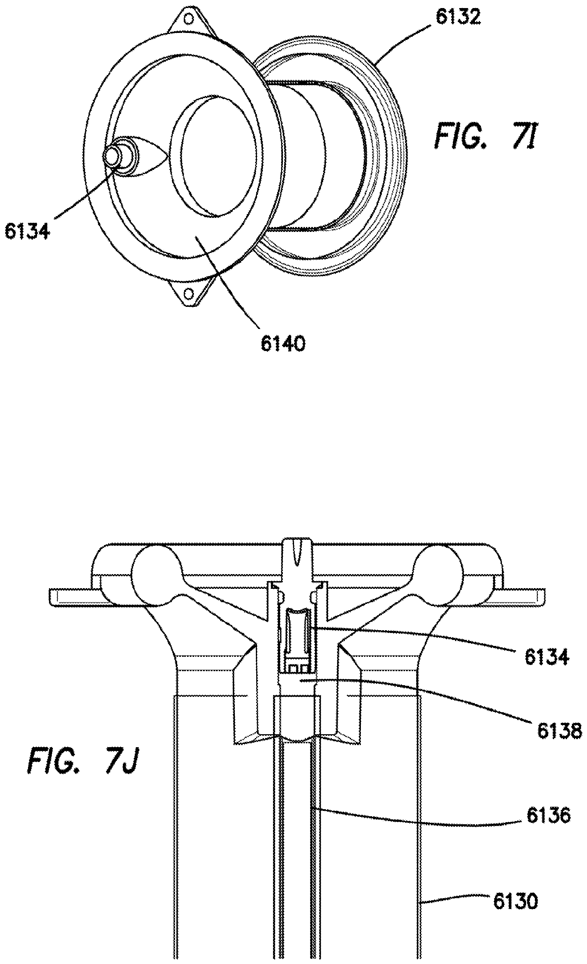

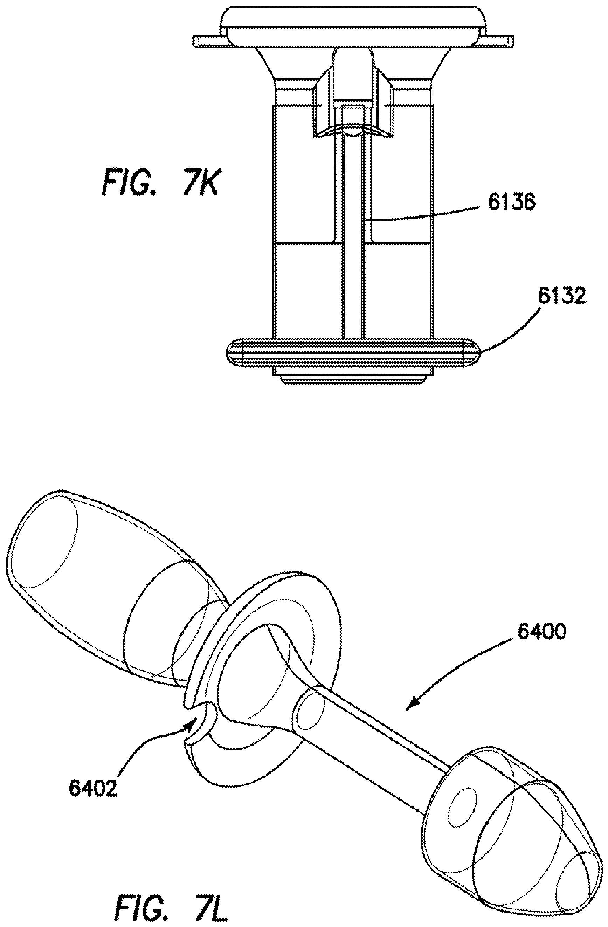

In use, the inflatable member may be inflated after the retractor is disposed within the natural orifice by inserting a syringe into the valve **6134** located at the proximal end **6138** of the channel within the tubular body (see FIG. **7I****).** As shown in FIGs. **7J** and **7K****,** the port leads into the channel **6136,** which allows the fluid or gas from the syringe to travel to the inflatable member **6132.** In this example, the optional obturator **6400** may be modified with an indent **6139** to provide clearance for the inflation port, as shown in **FIG. 7L****.**

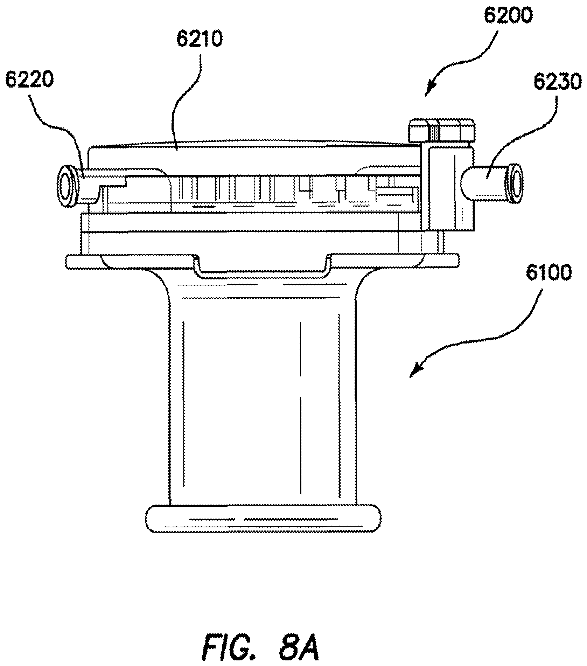

With reference to FIG. **8A****,** a side view of a natural orifice access device having a cap **6200** removably coupled to a retractor **6100** is illustrated. In the illustrated example, the cap **6200** comprises a sealable access surface **6210** such as a gel pad surface as described in further detail herein. In certain examples, the cap **6200** can also comprise at least one gas or fluid port **6220, 6230.** In the illustrated example, the cap **6200** comprises two gas or fluid ports **6220, 6230,** such that one port can be used for gas insufflation and the other port can be used for ventilation for example when electrosurgery is performed through the access device. In certain examples, at least one of the gas or fluid ports **6220, 6230** comprises a valve such as a stopcock valve to selectively control the flow of fluid there through.

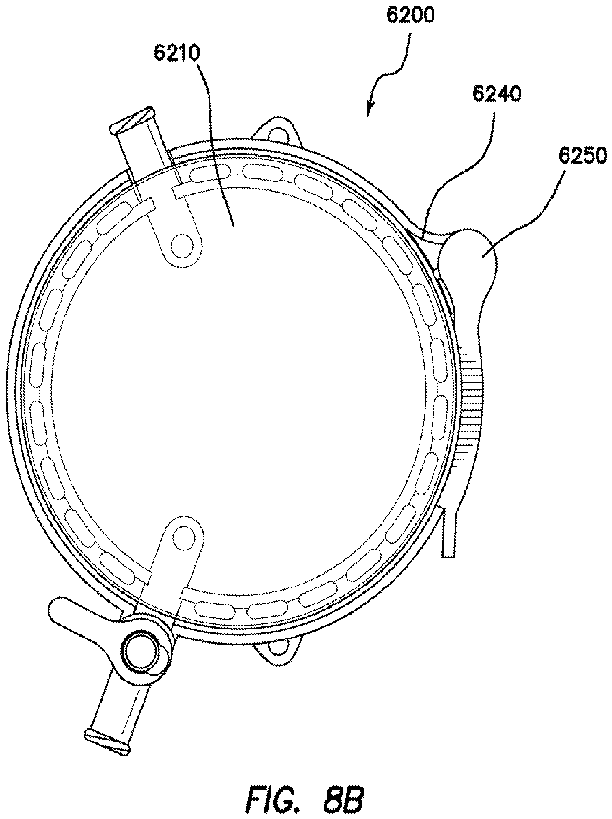

With reference to FIG. **8B****,** a top view of the natural orifice access device is illustrated. The sealable access surface **6210** can be encircled by and restrained by an annular frame **6240** such as a split ring having a clamp **6250.** The clamp **6250** can be movable between an open configuration in which the cap **6200** is selectively removable from the retractor **6100** and a clamped configuration in which the cap **6200** can be secured to the retractor **6100.** For example, the annular frame **6240** can be positioned peripherally around the outer ring **6120** with the clamp **6250** in the open configuration and the clamp moved to the clamped configuration to sealingly fix the cap **6200** to the retractor **6100.** Accordingly, the cap **6200** can be easily removed during a surgical procedure to facilitate removal of excised tissue from a surgical site through the retractor **6100.**

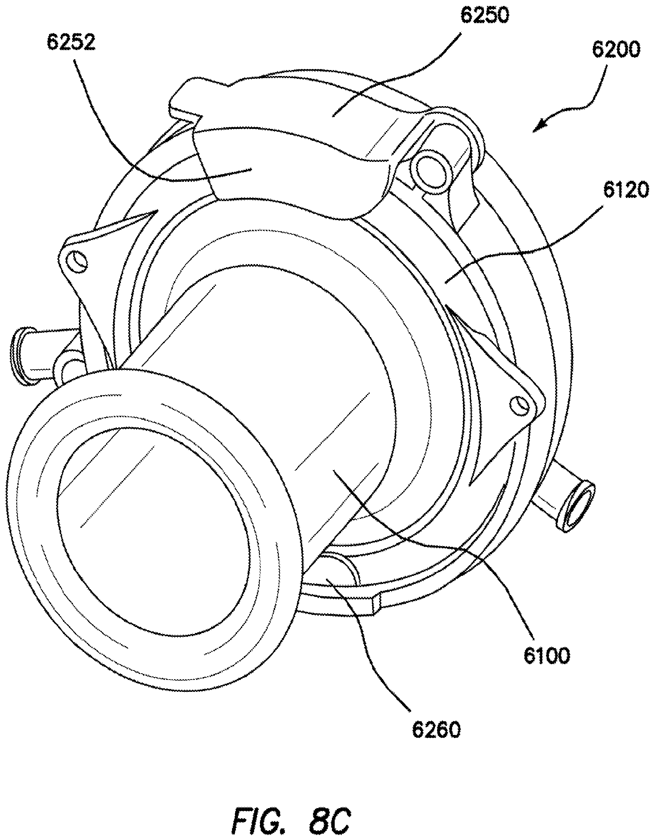

With reference to FIG. **8C****,** a perspective view of the natural orifice access device is illustrated. In the illustrated example, the clamp **6250** can have a distal flange **6252** positioned to interface with the outer ring **6120** of the retractor when the clamp is in the clamped configuration. As illustrated, the clamp **6250** engages a distal surface of the outer ring **6120** of the retractor **6100.** In some examples, the annular frame **6240** can further comprise at least one distal flange sized and positioned to interface with a retractor. In the illustrated example, the annular frame **6240** comprises a distal flange **6260** positioned to engage a distal surface of the outer ring **6120** of the retractor. As illustrated, the flange **6260** is generally diametrically opposed to the distal flange of the clamp **6250.** In other examples, the annular frame **6240** can include more than one distal flange positioned substantially equally spaced about the periphery of the annular frame **6240** or spaced irregularly about the periphery of the annular frame.

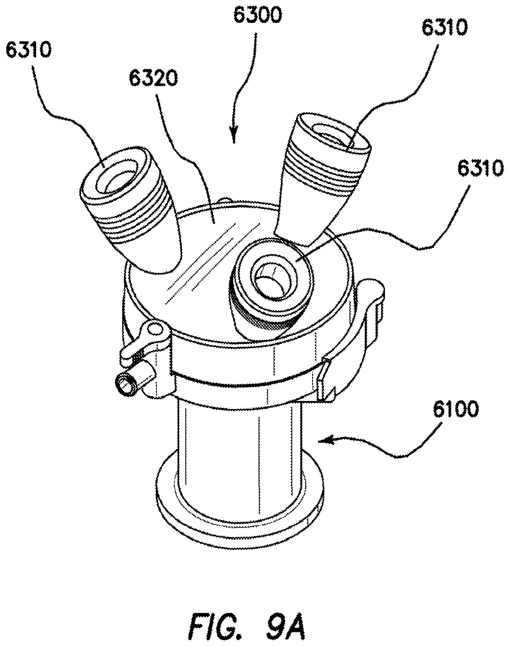

With reference to FIG. **9A****,** another example of natural orifice access device is illustrated with a cap **6300** removably coupled to a retractor **6100** such as that described above with respect to FIGS. **6A-6C**, 7A, 8A-8C, and **9A.** In the illustrated example, the cap **6300** includes multiple trocar access devices **6310** positioned through an access surface **6320** thereof. Advantageously, the multiple trocar access devices **6310** allow for easy placement and manipulation of multiple laparoscopic instruments in a surgical site through a single natural orifice.

In some examples, the inner ring **6110** and the outer ring **6120** independently have different footprint shapes and/or footprint diameters. For example, in the example illustrated in the example of retractor **7100** illustrated in FIGS. **6D**-F, 7B, 8D, and **9B,** the inner ring **7110** can be substantially flush with the tubular body **7130** while the outer ring **7120** can be an annular member having a generally circular cross-section. An inner ring **6110** with a larger diameter permits a greater retraction force, but is more difficult to insert and remove from a body cavity.

With reference to FIGS. **6D-6F****,** in some examples, a natural orifice access port or retractor **7100** can be adapted for use in a transanal endoscopic microsurgery (TEMS) procedure. The retractor **7100** comprises an inner or distal ring **7110,** an outer or proximal ring **7120,** a tubular body **7130,** and a funnel segment **7140** extending between and coupling the inner ring **7110** and the outer ring **7120.** The tubular body **7130** comprises a relatively flexible material such as a KRATON^{®} material or a silicone rubber material, which is substantially cylindrical in the illustrated example. In other examples, the tubular body **7130** has another shape, for example, an oval cross section. Some examples of the tubular body **7130** comprise one or more coatings that provide additional functionality, for example, an anti-microbial coating.

In the illustrated example, the inner ring **7110** is substantially flush with a distal end of the tubular body **7130** such that the retractor **7100** has a generally tubular configuration extending distally of the funnel segment **7140** to the distal end. Examples of the inner ring **7110** are sufficiently flexible and compliant to be compressed and/or deformed for insertion into a body orifice such as a patient's anus during a transanal surgical procedure. When subsequently released within an associated body cavity, the inner ring **7110** substantially returns to its original shape or footprint. In some examples, the inner ring **7110** assumes a substantially circular shape substantially flush with the generally cylindrical tubular body **7130** in a relaxed state, for example, when released within a body cavity. In other examples, the inner ring **7110** has another shape in the relaxed state, for example, an oval. The inner ring **7110** assumes a different shape when compressed for insertion through an incision or body orifice, for example, a substantially oval shape, a generally linear shape, a tear-drop shape, or another suitable shape. In other examples, the inner ring **7110** is substantially rigid, that is, non-compliant under the ordinary conditions under which it is used.

With continued reference to FIGS. **6D-6F****,** in some examples, the inner ring **7110** can be shaped and configured to facilitate insertion through a natural orifice. For example, in the illustrated example, the inner ring **7110** can include a radiused edge to facilitate atraumatic entry through a natural orifice. In other examples, the inner ring **7110** can include a beveled edge to facilitate entry through a natural orifice. Furthermore, in the illustrated example, the inner ring **7110** can be formed at an angle transverse to a longitudinal axis defined by the tubular body **7130.** Advantageously, such an angled inner ring **7110** can facilitate insertion of the retractor **7100** through a natural orifice. In other examples, the inner ring **7110** can be substantially perpendicular to the longitudinal axis defined by the tubular body.

With continued reference to FIGS. **6D-6F****,** the outer ring **7120** is proximal the funnel section **7140.** In the illustrated example, the outer ring **7120** has a substantially circular footprint. As further discussed herein, the outer ring **7120** can be sized and configured to sealingly couple to a cap or other access device thereon. In some examples, as discussed above with reference to the examples of FIGS. **6A-6C****,** one or more suture points can be disposed on the retractor **7100** adjacent the outer ring **7120.**

With continued reference to FIGS. **6D-6F**, the tubular body **7130** can have a generally circular profile defining a generally cylindrical passage **7150.** The generally cylindrical passage **7150** is desirably large enough to accommodate more than one laparoscopic instrument there through such that a single natural orifice access device can be used to provide access for multiple surgical instruments in a body cavity. Moreover, generally cylindrical passage **7150** is desirably large enough such that multiple surgical instruments positioned there through can be translated or pivoted relative to one another, allowing a surgeon to manipulate the instruments as desired during a surgical procedure. The generally cylindrical passage extends between a proximal end **7152** of the retractor **7100** adjacent the outer ring **7120** to a distal end **7154** of the retractor **7100** adjacent the inner ring **7110** (FIG. **6D****).**

With reference to FIG. **6D****,** in the illustrated example, the funnel segment **7140** provides a diametric reduction between the relatively large diameter of the outer ring **7120,** which is sized and configured to be removably coupled to an access device such as a cap, and the relatively smaller diameter of the passage **7150,** which is sized to fit within a natural orifice with minimal distention of the orifice. The funnel segment **7140** has an inner surface **7142** which can provide a bearing surface for an obturator used to advance to the retractor **7100** into a body cavity. In some examples, the funnel segment **7140** can have a substantially linear taper between the relatively large diameter and the relatively smaller diameter such that the inner surface **7142** is a frusto-conical segment. In other examples, the funnel segment **7140** can have a curved profile between the relatively large diameter and the relatively smaller diameter.

In some examples, a natural orifice access system can include a retractor **7100** and an optional obturator, such as described above with reference to FIG. **6G****.** The obturator can have a proximal bearing surface **6410** sized and configured to bear against the inner surface **7142** of the funnel segment **7140** and a distal dilation surface **6420** sized and configured to expand a natural orifice for passage of the retractor **7100.** Thus, during insertion of the retractor **7100** into a natural orifice, the dilation surface expands a pathway to a surgical site in a body cavity while the obturator bears on the inner surface **7142** of the funnel segment **7140** to advance the retractor **7100** into position in the surgical site. Furthermore, in some examples, the obturator can have a handle **6430** at a proximal end thereof adapted to facilitate selective twisting or rotation of the obturator about a longitudinal axis thereof during insertion.

In an alternative example, shown in FIG. **6I**, the obturator **6405** includes a straight shaft piece **6425** between the distal dilation surface **6420** and the proximal bearing surface **6410** that facilitates dilation of the natural orifice prior to inserting the retractor. It can then be combined with the retractor **7100** to help ease insertion, as shown in FIG. **6J****.**

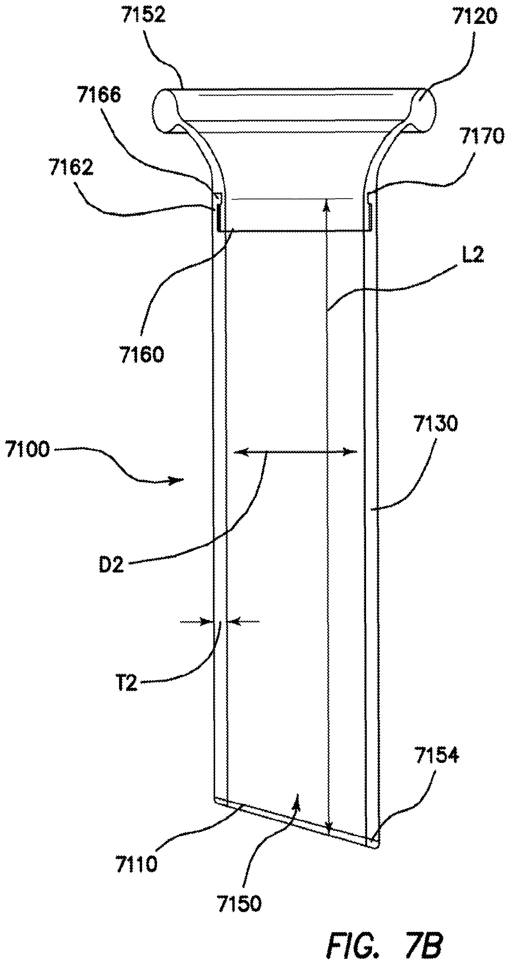

With reference to FIG. **7B****,** it can be desirable that the outer ring **7120** is relatively stiff compared with the relatively flexible tubular body **7130** of the retractor **7100** so that the outer ring **7120** can sealingly engage an access device such as a cap. In the illustrated example, the retractor **7100** is formed in a multiple-shot molding process. For example, in the illustrated example, an inner segment of the retractor **7100** defined by the tubular body **7130** and the inner ring **7110** is formed in one molding operation from a flexible material, and an outer segment of the retractor **7100** defined by the funnel segment **7140** and the outer ring **7120** is formed in another molding operation from a relatively rigid material such as a polycarbonate material or other suitable material.

In other examples, a multiple-shot molding process can be varied such that the resulting inner and outer segments are different from those of the illustrated example. For example, in certain examples, the inner segment can include the tubular body **7130,** the inner ring **7110,** and a portion of the funnel segment **7140,** while the outer segment can include a portion of the funnel segment **7140** and the outer ring **7120.** In certain other examples, the inner segment can include the inner ring **7110** and a portion of the tubular body **7130,** while the outer segment can include a portion of the tubular body **7130,** the funnel segment **7140,** and the outer ring **7120.**

With reference to FIGS. **6D** and **7B****,** a retractor **7100** formed in a multiple-shot molding process can include one or more retention members **7160** on the inner segment and the outer segment to maintain the position of the inner segment relative to the outer segment. For example, in some examples, a distal end of the outer segment can include one or more protrusions **7162** extending radially outwardly from the funnel segment **7140** and one or more recesses **7164** recessed radially inwardly from the funnel segment **7140** at an interface region of the inner segment and the outer segment of the retractor **7100.** In the illustrated example, the distal end of the outer segment includes a plurality of protrusions **7162** alternating with a plurality of recesses **7164** there between. Moreover, in some examples, the outer segment can include an annular groove **7170** formed in the funnel segment **7140** at an interface region of the inner segment and the outer segment of the retractor **7100.** The inner segment of the retractor **7100** can include an annular member **7166** disposed within and matingly engaging the groove **7170** to maintain the position of the inner segment relative to the outer segment.

With reference to FIG. **7B****,** a cross-sectional view of retractor **7100** is shown. In the illustrated example, the tubular body **7130** is formed of a flexible material having a predetermined fixed length L2, inner diameter D2, and wall thickness T2. The fixed length L2, inner diameter D2, and wall thickness T2 are selected to accommodate the anatomy of a natural orifice, such as the anal orifice of a majority of patients. It is contemplated that the retractor **7100** can be scaled to different sizes for patients of different ages. Furthermore, in some examples, it is contemplated that the retractor can include a telescopic tubular body such that the tubular body can be selectively positioned at a variety of lengths depending on patient anatomy and the location of the surgical site within the body cavity. Desirably, the wall thickness T2 and material of the tubular body **7130** are selected such that the tubular body **7130** is resilient enough to maintain the passage **7150** there through when positioned in the natural orifice. Moreover, desirably, the inner diameter, D2 is sufficiently large to accommodate multiple surgical instruments. For example, in examples of the retractor **7100** adapted for use in a transanal surgical procedure, the inner diameter D2 and thickness T2 can be sized such that an outer diameter of the retractor can be between approximately 30 mm and 70 mm, desirably between approximately 35 mm and 50 mm, and in one example approximately 40 mm. Additionally, desirably, the fixed length L2 is sufficiently long such that the inner ring **7110** can be positioned at a surgical site within a body cavity and the outer ring **7120** can be positioned outside the natural orifice. In some examples, the fixed length L2 is of a length such that the device has an overall length between the proximal end **7152** and the distal end **7154** of between approximately 100 mm and approximately 200 mm, desirably between approximately 120 mm and 180 mm, more desirably between approximately 140 mm and 160 mm, and in one example, approximately 150 mm.

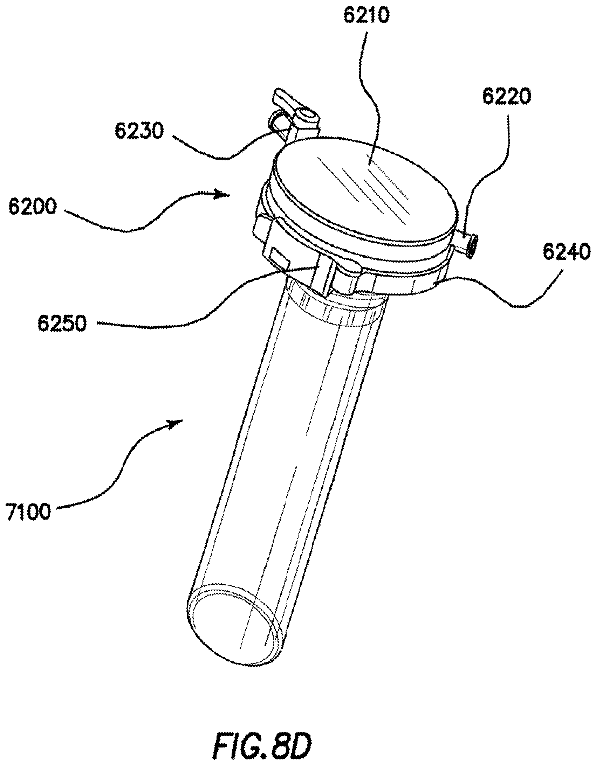

With reference to FIG. **8D****,** a perspective view of a natural orifice access device having a cap **6200** substantially similar to that described with respect to FIGS. **8A-8C** removably coupled to a retractor **7100** is illustrated. In the illustrated example, the cap **6200** comprises a sealable access surface **6210** such as a gel pad surface as described in further detail herein. In certain examples, the cap **6200** can also comprise at least one gas or fluid port **6220, 6230.** In the illustrated example, the cap **6200** comprises two gas or fluid ports **6220, 6230,** such that one port can be used for gas insufflation and the other port can be used for ventilation for example when electrosurgery is performed through the access device. In certain examples, at least one of the gas or fluid ports **6220, 6230** comprises a valve such as a stopcock valve to selectively control the flow of fluid there through.

With continued reference to FIG. **8D****,** a top view of the natural orifice access device is illustrated. The sealable access surface **6210** can be encircled by and restrained by an annular frame **6240** such as a split ring having a clamp **6250.** The clamp **6250** can be movable between an open configuration in which the cap **6200** is selectively removable from the retractor **7100** and a clamped configuration in which the cap **6200** can be secured to the retractor **7100.** For example, the annular frame **6240** can be positioned peripherally around the outer ring **7120** with the clamp **6250** in the open configuration and the clamp moved to the clamped configuration to sealingly fix the cap **6200** to the retractor **7100.** Accordingly, the cap **6200** can be easily removed during a surgical procedure to facilitate removal of excised tissue from a surgical site through the retractor **7100.**

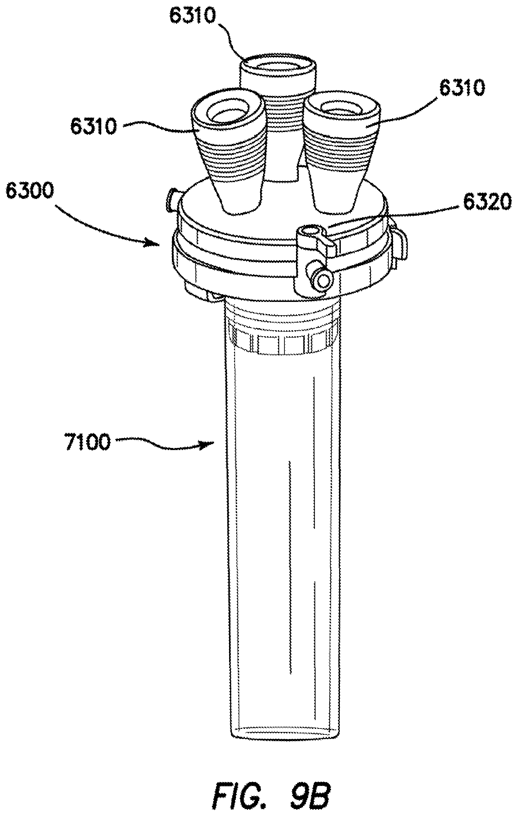

With reference to FIG. **9B****,** another example of natural orifice access device is illustrated can include a cap **6300** substantially similar to that described above with reference to FIG. **9A** removably coupled to a retractor **7100** such as that described above with respect to FIGS. **6D**-F, 7B, and **8D.** The cap **6300** can include multiple trocar access devices **6310** positioned through an access surface **6320** thereof. Advantageously, the multiple trocar access devices **6310** allow for easy placement and manipulation of multiple laparoscopic instruments in a surgical site through a single natural orifice.

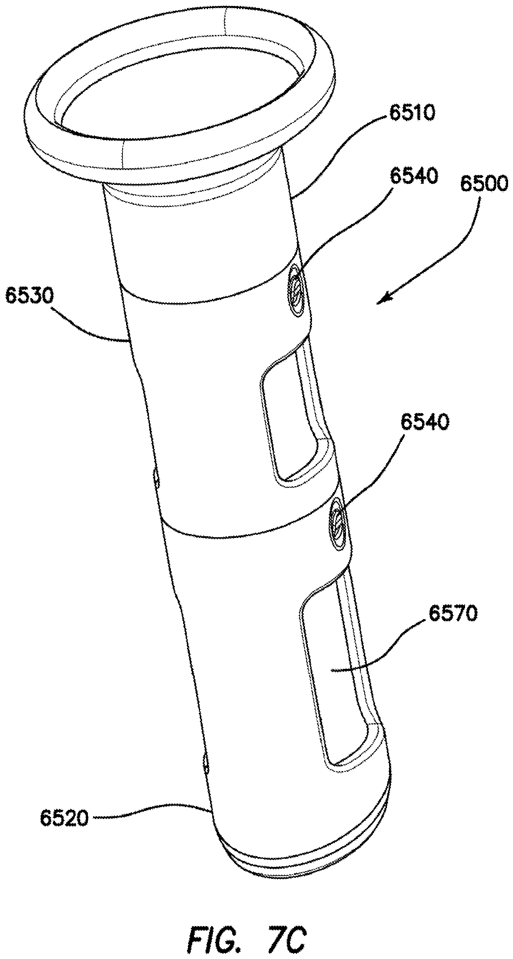

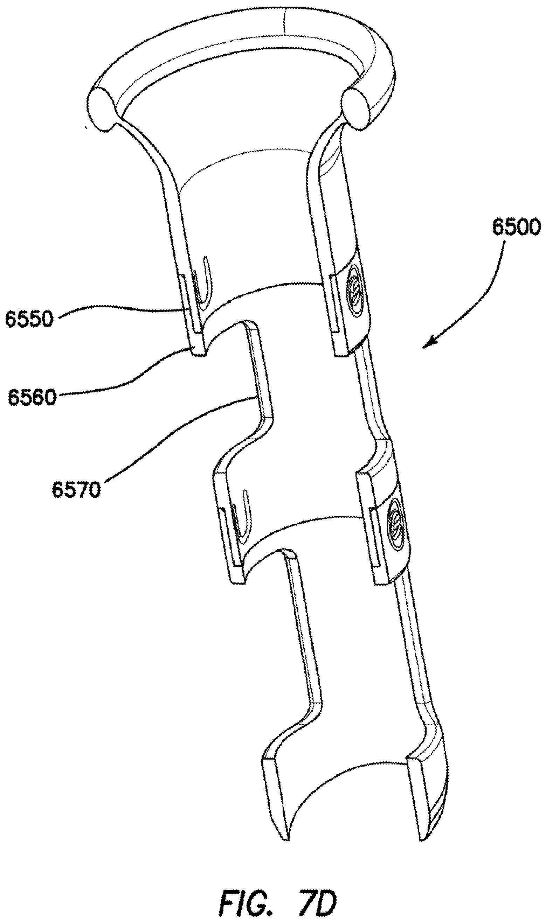

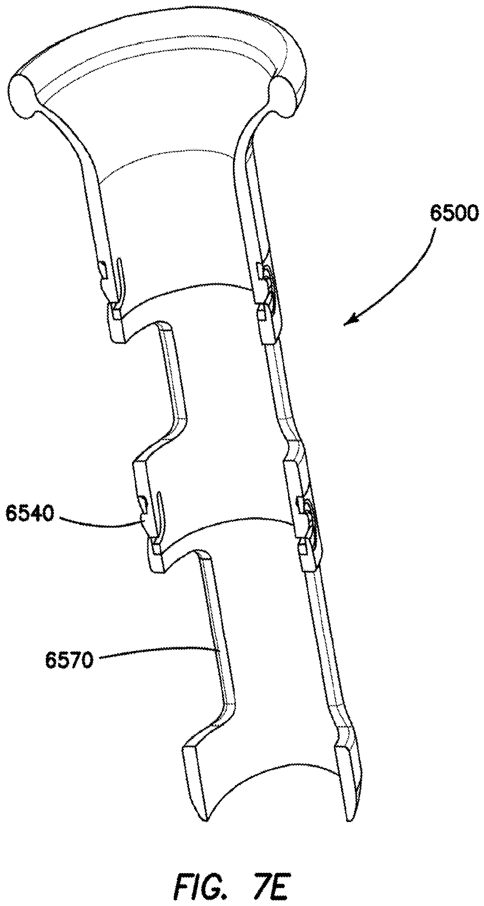

As discussed herein, the retractors shown in FIGs. **7A** and **7B** can include a telescopic tubular body such that the tubular body can be selectively positioned at a variety of lengths depending on patient anatomy and the location of the surgical site within the body cavity. In another example, illustrated in FIG. **7C****,** the tubular body may be formed in sections of varying length that slidingly engage and snap lock together to provide a variety of lengths, depending of the number and size of the sections selected and assembled. With reference to FIG. **7C****,** a perspective view of a retractor **6500** is shown having three sections: an outer ring section **6510,** an inner ring section **6520,** and an intermediate section **6530** disposed between the other two sections. The three sections are held together by a snap lock mechanism **6540.** Each section terminates at the distal end with an annular groove **6550** that slidingly engages with the proximal end **6560** of the next section, best shown in the cross section view of FIG. **7D****.** The snap lock mechanism is shown in cross-section in FIG. **7E****.** The tubular body of the example shown in FIG. **7C**-E is preferably formed from a relatively stiff material, such as a polycarbonate.

Optionally, as shown in FIG. **7C-7F****,** the tubular body of the retractor can include cut-out portions or windows **6570,** to provide access to regions of the anatomy that would otherwise be obscured by the tubular body while the retractor is in place. Thus, the retractor can be inserted into the body orifice or incision to provide retraction and to protect the lining of the body cavity, and then manipulated to align the window(s) to the sites of interest in the body cavity for access by surgical instruments.

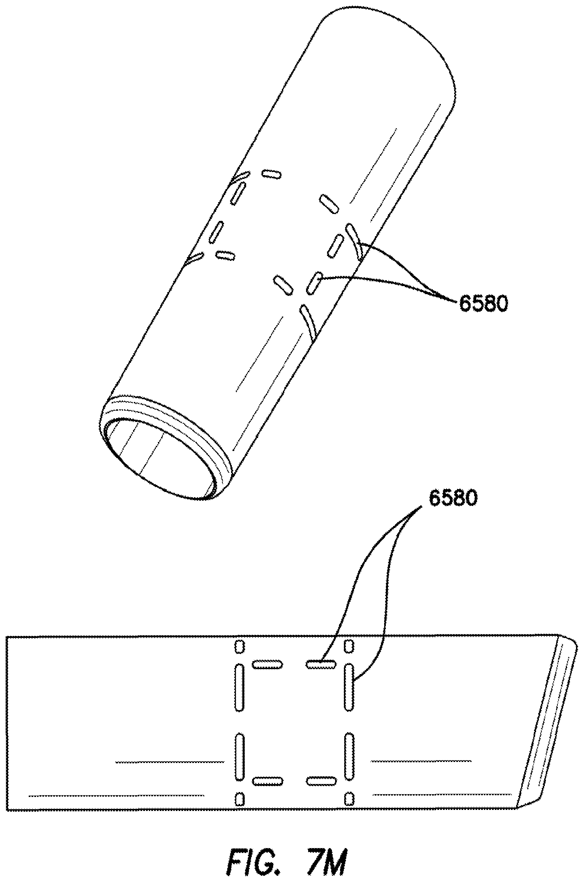

As will be appreciated, such cut-out portions may be provided in retractors having tubular bodies of both rigid and flexible construction, as well as tubular bodies formed as a single piece or in sections. FIG. **7M** shows an example of a flexible tubular body of a retractor, both in side view and in perspective view, wherein the tubular body contains perforations **6580.** The tubular body can be cut or torn at the perforations to vary the length of the tubular body and/or to incorporate cut-out portions into the tubular body. The tubular body of the example shown in FIG. **7M** is preferably formed from a relatively flexible material, such as KRATON^{®} or PELLETHANE^{®}.

In the illustrated examples of FIGs. **9A** and **9B****,** the trocar access devices **6310** have a relatively low profile, that is, protrude minimally above the access surface **6320** and/or below the distal surface of the cap **6300.** Accordingly, the trocar access devices **6310** are shorter than a length of a typical trocar and comprise a seal assembly positioned above the access surface **6320** and a cannula extending through the gel pad of the cap **6300.** The reduced length of the trocar access devices **6310** allows increased angular or pivotal motion for instruments extending there through, and also permits the use of curved and/or angled instruments.

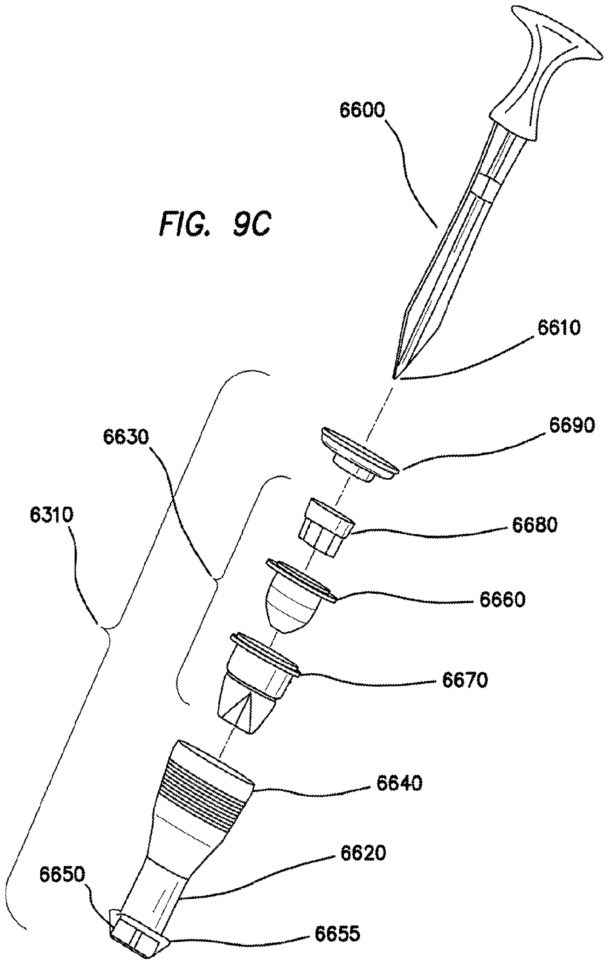

FIG. **9C** is an exploded view of an example of a trocar access device **6310** and optional obturator **6600,** which is a component of some examples of the access device system. In the illustrated example, the obturator **6600** comprises a pointed, puncture tip **6610.**

The trocar access device **6310** comprises a proximal end, a distal end, and a longitudinal axis. The trocar access device **6310** comprises a cannula **6620** extending along the longitudinal axis. A trocar seal **6630** is disposed at the proximal end of the cannula **6620,** contained within a housing **6640.** A retainer **6650** is disposed at the distal end or tip of the cannula **6620.**

The cannula **6620** comprises a tubular body dimensioned to accommodate an instrument or instruments received there through. In the illustrated example, the cannula **6620** is a substantially cylindrical tube, and extends through the cap **6300** in use. In the illustrated example, the cannula **6620** is comparatively short because the cannula need only traverse the cap 6300 (FIG. **9A**-B), which has a known and consistent thickness, rather than a body wall. Accordingly, some examples of the cannula **6620** are not more than about 2-times longer, about 1.5-times longer, about 1.2-times longer, or about 1.1-times longer than the thickness of the gel pad. In some examples, the cannula **6620** is less than about 20 mm, about 10 mm, or about 5 mm longer than the thickness of the gel pad. In some examples, the cannula **6620** is about as long as the gel pad is thick. In other example, the cannula **6620** has a different length, for example, a length typical for a cannula used for traversing a body wall. Shorter length cannula permit increased angular degrees of freedom for instruments passing there through. Examples of shorter cannula also accommodate curved instruments. The cannula **6620** comprises any suitable biocompatible material. In some examples, the cannula **6620** comprises a flexible material.

The illustrated trocar seal **6630** comprises an instrument or septum seal **6660** and a zero seal **6670.** Optionally, a shield **6680** may be disposed within the instrument seal **6660.** The instrument seal **6660** seals instruments passing there through, thereby maintaining pressurization in a body cavity such as pneumoperitoneum or pneumorectum. The zero seal **6670** provides a seal when no instrument passes through the trocar seal **6630.** The instrument seal **6660** and zero seal **6670** are received in a housing **6640** disposed at the proximal end of the cannula **6620** and secured therein by a seal cover **6690.**

The retainer **6650** is disposed at or near the distal end of the cannula **6620.** In some examples, the retainer **6650** and cannula **6630** are integrated, while in other examples, the retainer **6650** and cannula **6630** are not integrated. In the illustrated example, the proximal end of the retainer **6650** comprises a flange **6655** that is generally flat and perpendicular to the longitudinal axis, while the distal end is tapered, narrowing toward the distal end of the cannula **6620.** The flange **6655** reduces the likelihood of accidental or inadvertent removal of the trocar access device **6310** from the cap. Some examples of the proximal face of the flange **6655** comprise additional anchoring features, for example, at least one of barbs, spikes, ridges, texturing, and the like, which are configured to penetrate or bite into a distal face of the cap **6300.** In some examples, a diameter of the flange **6655** is from about 1.2 to about 2.5 times wider, or from about 1.5 to about 2.0 times wider than an outer diameter of the cannula **6630.** Some examples of the trocar access device **6310** are 5-mm trocars, in which the outer diameter of the cannula **6620** is from about 7 mm to about 8 mm.

The tapered end of the retainer **6650** facilitates insertion of the trocar access device **6310** through the cap, either by itself, or when assembled with the obturator **6600** extending there through. For example, in some examples, the retainer **6650** is inserted through a preformed opening in the cap **6300.**

In some examples in which the retainer **6650** and cannula **6620** are not integrated, that is, are separate components, the retainer **6650** is secured to the cannula **6620** after the cannula **6620** is inserted through the cap. In some examples, the cannula **6620** and retainer **6650** are secured mechanically, for example, using latches, screw threads, clips, lock rings, ratchets, and the like. In some examples, the cannula **6620** and retainer **6650** are secured adhesively. In some examples, the position of the retainer **6650** is adjustable, for example, to accommodate caps of different thicknesses. In some examples, the cannula **6620** and/or retainer **6650** is secured to the cap, for example, adhesively.

An example of a procedure for retracting a body orifice is described with reference to the examples of the retractor **6100** illustrated in FIGS. **6A-6C**, 7A, 8A-8C, and **9A,** and the examples of retractor **7100** illustrated in FIGS. **6D-6F**, 7B, 8D, and **9B,** although the procedure is applicable to all of the examples of the retractor disclosed herein. In use, the surgical wound retractor **6100, 7100** is inserted into a body orifice, such as the vagina (FIG. **2****),** mouth (FIG. **3****)** or anus (FIG. **4****).** The inner ring **6110, 7110** is folded or compressed into an oval or other suitable shape and urged through the incision or body orifice into an associated body cavity. Once the inner ring **6110, 7110** is fully disposed within the associated body cavity, it is allowed to resume its original, relaxed shape, for example, substantially circular, oval, or other original shape. In some examples, the inner ring **6110** is then pulled upward against the inner surface of the body cavity, for example, by pulling the outer ring **6120** upward. An outer surface of the tubular body **6130, 7130** retracts the natural orifice.

As illustrated in FIG. **5****,** some examples of the access device **5000** comprise a cap, cover, or lid **5500** coupled to the outer ring of the retractor **5100,** which seals the retractor **5100,** for example, for maintaining pressurization within a body cavity such as pneumoperitoneum or pneumorectum. In some examples, lid **5500** is removable, for example to provide access into the body cavity. Some examples of the lid **5500** comprise a transparent or translucent portion, thereby allowing a user to view into the body cavity without removing the lid **5500.** As will be described below, one example of a lid **5500** is a gel cap. In some examples, a cross-sectional shape of the outer ring **6120** (FIG. **6A****), 7120** (FIG. **6D****)** of the retractor is selected to reduce or prevent the lid **5500** from partial and/or incorrect coupling to the outer ring **6110** (FIG. **6A****), 7120** (FIG. **6D****)** of the wound retractor. Such cross-sectional shapes include oval and rectangular, or any other suitable cross-sectional shape that provides the desired functionality, for example, hexagonal, octagonal, and the like. Additionally, depending on the use and on surgeon preference, in some examples, each of the inner ring **6110, 7110** and outer ring **6120, 7120** of the wound retractor includes independently variable design configurations. For example, examples of the inner ring **6110, 7110** and/or the outer ring **6120, 7120** are rigid or flexible, and have footprints, cross-sectional shapes, and/or dimensions dependent on the intended use, for example, circular or oval footprints, diameters dependent on incision or orifice dimensions, or cross-sectional dimensions dependent on retraction force. In some examples, the inner ring **6100** may extend radially out from the tubular body **6130** when deployed, stabilizing the retractor within the body orifice (FIG. **7A****).** In other examples, the inner ring **7110** may be flush with the tubular body **7130,** as where, for example, the length L2 of the tubular body is sufficient to stabilize the retractor within the body orifice (FIG. **7B****).**

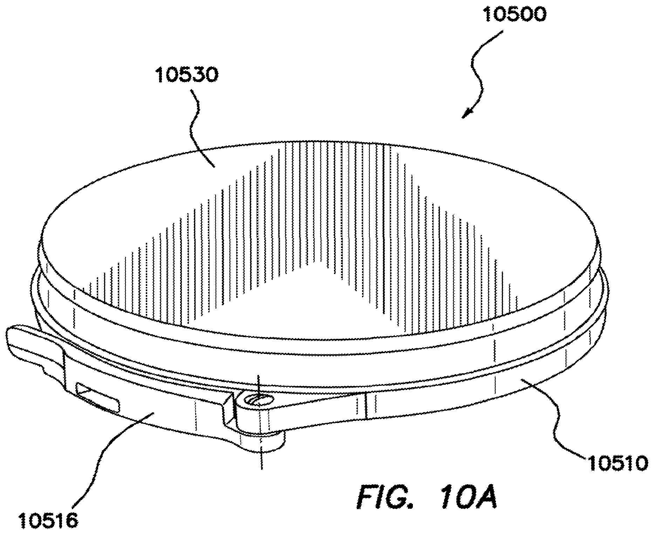

FIG. **10A** illustrates in perspective an example of a cap or cover **10500,** which is a surgical access device that seals the opening between the body cavity and the area outside the body cavity while providing access into the body cavity from outside the body cavity. More particularly, the illustrated cap **10500** releasably and sealingly couples to the outer ring **6120** (FIG. **6A****), 7120** (FIG. **6D****)** of the wound retractor. The cap **10500** comprises a cap ring **10510** dimensioned and configured for coupling to the outer ring **6120, 7120** of the wound retractor and a pad **10530** coupled to the cap ring **10510.** Examples of the cap **10500** provide an artificial body wall with consistent properties compared with a natural body wall, for example, thickness, compliance, rigidity, uniformity, and the like.

The illustrated cap or cover **10500** is substantially circular. In other example, the gel cap **10500** has another shape or footprint, for example, oval, elliptical, parabolic, square, rectangular, or another suitable curved or polygonal shape. In some examples, the outer ring **6120, 7120** of the retractor and cap ring **10510** of the cap have the same general shape or footprint. In other examples, the outer ring **6120, 7120** of the retractor and cap ring **10501** of the cap have substantially different shapes, for example, a generally circular outer ring **6120, 7120** and an oval cap ring **10510.** In these examples, the outer ring **6120, 7120** is distorted or reshaped for coupling to the cap ring **10510,** for example, by compressing opposed sides of the outer ring **6120, 7120.** Non-circular shapes are useful, for example, for procedures in which space is limited. As discussed above, retracting a long, straight incision using an oval or elongated retractor requires less force than a similar procedure using a circular retractor.

In some examples, the pad **10530** comprises a gel. In such examples, the pad **10530** is referred to as a "gel pad" and the cap **10500** is referred to as a "gel cap". Descriptions of gel pads and gel caps generally apply to examples in which the pad **10530** does not comprise gel unless otherwise specified. In some examples, the gel pad **10530** does not comprise any preformed access channels there through, for example, for instrument access. Instruments may be inserted directly through the gel pad **10530,** puncturing the gel pad **10530,** and thereby creating access channels or portions in the gel pad **10530.** Each access portion forms an instrument seal in the presence of an instrument inserted there through and a zero seal in the absence of an instrument inserted there through. The gel provides a gas tight seal around a variety of shapes and sizes of instruments inserted there through. Some examples of the gel pad **10530** also provide trocar access directly there through, which also provide instrument access into the body cavity. Examples of the gel pad **10530** have a working diameter of from about 40 mm to about 120 mm, which is the diameter of a portion of the gel pad **10530** through which instruments and/or trocars may be inserted. Examples of the gel cap **10500** are typically from about 10 mm to 50 mm wider than the working diameter.

Accordingly, examples of the gel cap **10500** maintain pressurization within a body cavity such as pneumoperitoneum or pneumorectum during multiple instrument exchanges and substantially prevent unintentional loss of pressurization. Examples of the gel cap **10500** also provide substantially continuous access and visibility during surgery. Examples of the gel cap **10500** have a small profile for use in procedures with limited surgical space.

In some examples, the gel is an ultragel, which is characterized by an ultimate elongation greater than about 1000 percent and a durometer less than about 5 Shore A. Some examples of the ultragel comprising KRATON^{®} and mineral oil exhibit an ultimate elongation exceeding about 1500 percent and improved sealing properties, for example, sealing with instruments of a wider size range than other seal materials. In some examples, the seals comprising ultragels also form zero seals when the instrument is removed therefrom. Accordingly, in some examples of seals comprising ultragels, a single seal is acts as both the instrument seal as well as the zero seal.

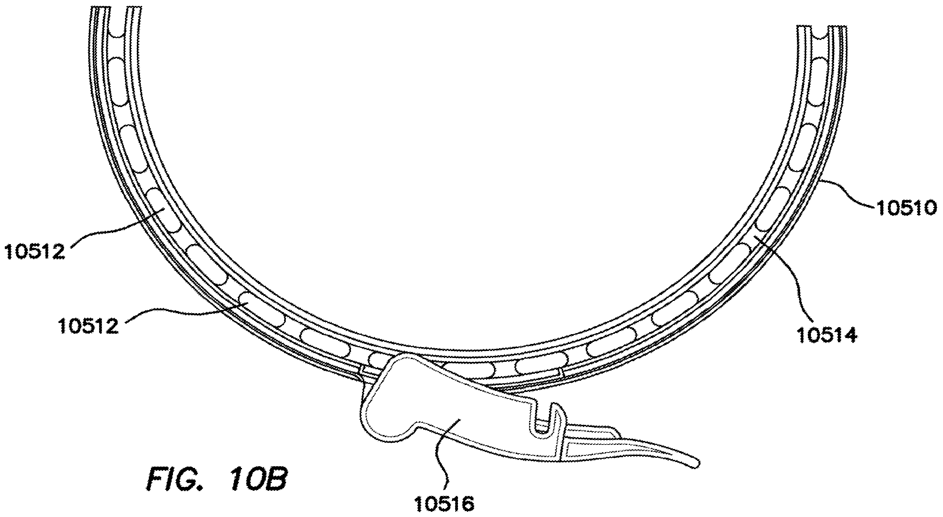

Some examples of the cap ring **10510** comprise a substantially cylindrical ring comprising a proximal portion, a distal portion, and a longitudinal axis extending from the proximal portion to distal portions. In other examples, the cap ring **10510** has another shape or footprint, for example, oval. As best seen in FIG. **10B****,** which is a bottom view of a cap ring **10510,** in the illustrated example, the proximal portion of the cap ring **10510** comprises a plurality of apertures **10512** distributed about the periphery thereof. The apertures **10512** extend through a wall **10514** at the proximal portion of the cap ring. In other examples, the apertures **10512** are disposed in at least one member extending either longitudinally inward or longitudinally outward from the wall **10514** of the cap ring. The gel pad **10530** is disposed at the proximal portion of the cap ring **10510** in the illustrated example, with portions of the gel pad **10530** extending through the apertures **10512,** thereby creating an interlocking structure between the cap ring **10510** and the gel pad **10530,** mechanically locking the cap ring **10510** and the gel pad **10530** together.

The distal portion of the cap ring **10510** is substantially cylindrical in the illustrated example, and is dimensioned and configured to receive the outer ring **6120** (FIG. **6A****), 7120** (FIG. **6D****)** of the wound retractor. The cap ring **10510** comprises a latch mechanism **10516** that removably couples the cap ring **10510** to the outer ring **6120, 7120.** Those skilled in the art will understand that other mechanisms are also useful for coupling the cap ring **10510** to the outer ring **6120, 7120** of the wound retractor, for example, protruding lips, levers, clips, latches, tongues, grooves, screw threads, bayonet mounts, screws, friction fittings, compression fitting, snap caps, and the like. In the illustrated example, when the outer ring **6120, 7120** of the wound retractor is received in the distal portion of the cap ring **10510,** the outer ring **6120, 7120** of the wound retractor contacts and embeds within a portion of the gel pad **10530** disposed at the distal portion of the cap ring **10510,** thereby displacing a portion of the gel, and forming a seal between the gel pad **10530,** and the outer ring **6120, 7120** and tubular body **6130, 7130** of the wound retractor. Thus, the distal portion of the gel pad **10530** is in juxtaposition with the incision or body orifice. In other examples, the cap ring **10510** is permanently coupled or fixed to the outer ring **6120, 7120.**

The cap ring **10510** in some examples comprises a polymer. Examples of suitable polymers include, at least one of polyethylene (PE), low density polyethylene (LDPE), high density polyethylene (HDPE), ultra high molecular weight polyethylene (UHMWPE), polycarbonate, thermoplastic elastomers (DYNAFLEX^{®}, GLS Corp.; KRATON^{®}, Kraton Polymers), polyphenylene oxide (PPO), polystyrene, and the like. The polymer component of the cap ring is fabricated by any suitable method, including injection molding, melt casting, blow molding, and the like.

Some examples of a process in which the gel pad **10530** is cast in the cap ring **10510** are include steps performed at temperatures above about 130 °C over several hours, for example, from about three (3) to about four (4) hours. Accordingly, in some of these examples, the cap ring **10510** does not deform under these conditions.

Some examples of the gel pad **10530** comprise an elastomeric gel. Examples of the gel are prepared by mixing at least one triblock copolymer with a solvent that dissolves the midblocks of the triblock copolymer. The mixture is typically a slurry. The endblocks typically comprise a thermoplastic material, such as styrene, while the midblocks typically comprise a thermoset elastomer such as, ethylene/butylene, isoprene, or butadiene. Examples of the triblock copolymer include styrene-ethylene/butylene-styrene (SEBS), styrene-isoprene-styrene (SIS), and styrene-butadiene-styrene (SBS). In some examples, the solvent is an oil, for example, mineral oil. Upon heating a mixture or slurry of the triblock copolymer, the midblocks dissolve in the mineral oil, thereby forming a network of the insoluble endblocks. The resulting network has enhanced elastomeric properties compared with the parent copolymer. In some examples, the triblock copolymer used is KRATON^{®} G1651, which has a styrene to rubber ratio of 33/67. Once formed, the gel is substantially permanent and, by the nature of the endblocks, processable as a thermoplastic elastomer henceforward. The mixture or slurry has a minimum temperature at which it becomes a gel, which is referred to as the minimum gelling temperature (MGT). This temperature typically corresponds to the glass transition temperature of the thermoplastic endblock plus a few degrees. For example, the MGT for a mixture of KRATON^{®} G1651 and mineral oil is about 120 °C. When the slurry reaches the MGT and the transformation to a gel state takes place, the gel becomes more transparent, thereby providing a visual endpoint confirming the complete transformation of the slurry to the gel state, whereupon the gel may be cooled. Some examples of the gel comprise a diblock copolymer, either instead of or in addition to the triblock copolymer. Examples of the diblock copolymer comprise a thermoplastic first endblock, for example, styrene, and a thermoset elastomeric second endblock, for example, ethylene/butylene, isoprene, or butadiene. An example of a suitable diblock copolymer is styrene-ethylene/butylene (SEB).

For a given mass of slurry to form a complete gel, the entire mass of the slurry is heated to or above the MGT and held at or above the MGT for a sufficient time for the end blocks to form a network or matrix of interconnections. The slurry will continue to form a gel at temperatures between the MGT and temperatures at which the components of the slurry/gel begin to decompose and/or oxidize. For example, when the slurry/gel is heated at temperatures above 250 °C, the mineral oil in the slurry/gel will begin to be volatile and oxidize. Oxidizing may cause the gel to turn brown and become oily.

The speed at which a given volume of slurry forms a gel depends on the speed with which the entire mass of slurry reaches the MGT. Also, at temperatures higher than the MGT, the end block networks distribute and form more rapidly, thereby speeding the gel formation.

The various base gel formulas may also be mixed or alloyed with one another to provide gels with a variety of intermediate properties. For example, KRATON^{®} G1701X is a mixture of seventy percent (70%) SEB and thirty percent (30%) SEBS, with an overall styrene to rubber ratio of 28/72. Those skilled in the art will appreciate that an almost unlimited number of combinations, alloys, and styrene to rubber ratios can be formulated, each providing an example exhibiting one or more advantages, for example, low durometer, high elongation, and good tear strength.

Some examples of the gel material further comprise a polymer that, with a foaming agent, improves the sealing properties of the gel, for example, silicone, soft urethanes, and even harder plastics. Examples of suitable silicones include those used for electronic encapsulation. Examples of suitable harder plastics include polyvinylchloride (PVC), isoprene, KRATON^{®} neat, and other KRATON^{®}/oil mixtures. In the KRATON^{®}/oil mixture, suitable oils include vegetable oils, petroleum oils, and silicone oils, as well as mineral oil.

Some examples of the gel comprise one or more additives that provide one or more desirable properties, for example, at least one of enhanced lubricity, improved appearance, and wound protection. Additives are incorporated directly into the gel and/or applied as a surface treatment. In some examples, other compounds are added to the gel to modify its physical properties and/or to assist in subsequent modification of the surface by providing bonding sites and/or surface charges. Additionally, oil-based colorants are added to the slurry to create gels of different colors in some examples.

Some examples of the gel pad **10530** comprise a layer of polyethylene on at least one surface. Polyethylene is dissolved in mineral oil and the solution applied to one or more surfaces of the gel pad **10530.** The mineral oil does not evaporate, but instead, absorbs into the gel pad over time, leaving behind the polyethylene as a layer on the surface of the gel pad.

In some examples, the triblock copolymer/solvent mixture/slurry used to manufacture the gel pad **10530** comprises about ninety percent (90%) by weight of mineral oil and about ten percent (10%) by weight of KRATON^{®} G1651. From a thermodynamic standpoint, this mixture behaves similarly to mineral oil. Because mineral oil has a relatively high heat capacity, transforming 0.45 kg (1 pound) of the slurry into a homogenous gel at about 130 °C may take from bout three (3) to about four (4) hours. Once formed, the gel can be cooled as quickly as practicable with no apparent deleterious effects on the gel. In some examples, the gel is cooled by cold-water immersion. In other examples, the gel is air-cooled. Those skilled in the art will recognize that other cooling techniques are used in other examples.

Certain properties of the KRATON^{®}/oil gel will vary with the weight ratio of the components. In general, a higher proportion of mineral oil results in a softer gel, while a higher proportion of KRATON^{®} results in a firmer gel. A too-soft gel exhibits excessive tenting or doming of the gel cap **10500** during surgery when a patient's body cavity is insufflated. Some examples of gels that are too soft also do provide an adequate instrument seal and/or zero seal. The gel should be sufficiently soft to provide an adequate seal both in the presence of an instrument and in the absence of an instrument, however.

On prolonged or extended sitting or standing, the copolymer, such as KRATON^{®}, and the solvent, such as mineral oil, in the slurry may separate. The slurry may be mixed to greater homogeneity, for example, with a high shear mixer. Mixing the slurry may introduce or add air to the slurry, however. To remove air from the slurry, the slurry may be degassed. In some examples, the slurry is degassed under a vacuum, for example, within a vacuum chamber. In some examples, the applied vacuum is about 0.79 meters (about 29.9 inches) of mercury, or about one (1) atmosphere. Optionally, stirring or mixing the slurry under vacuum facilitates removal of the air. 1 inch of mercury = 3.39 kPa.

During degassing under vacuum, the slurry typically expands, then bubbles, and then reduces in volume. The vacuum is typically discontinued when the bubbling substantially ceases. Degassing the slurry in a vacuum chamber reduces the volume of the slurry by about ten percent (10%). Degassing the slurry also reduces oxidation of the finished gel in some examples.

Degassing the slurry tends to result in a firmer gel. A gel made from a degassed slurry comprising about 91.6% by weight of mineral oil and about 8.4% by weight of KRATON^{®} G1651, an eleven-to-one ratio, has about the same firmness as a gel made from a slurry that is not degassed and that comprises about ninety percent (90%) by weight of mineral oil and about ten percent (10%) by weight of KRATON^{®} G1651, a nine-to-one ratio.

Because mineral oil typically has a lower density than KRATON^{®}, the two components will separate after mixing, with the less dense mineral oil rising to the top of the container. This phase separation typically occurs when transforming a static slurry into a gel over several hours. Consequently, the resulting gel is non-homogeneous, with a higher concentration of mineral oil at the top and a lower concentration at the bottom. The speed of separation is a function of the depth or head height of the slurry being heated. Factors relevant to the relative homogeneity of the gel include the mass of slurry, the head height, the temperature at which the gel sets, and the speed at which the energy is transferred to the gel.

The gel pad **10530** or gel cap **10500** are gamma sterilized in some examples, which is relatively and/or comparatively simpler to qualify compared with other sterilization process, for example, versus ethylene oxide. Gamma sterilization can cause large bubbles to form in the gel pad, however, which are cosmetic and/or aesthetic issues in the sterilized devices. Because bubbles typically comprise greater than ninety-nine percent (99%) room air, the dissolved air is advantageously removed from the slurry prior to transforming the slurry into a gel. For example, the slurry may be degassed under vacuum, as described above, then gelled by heating. Some bubbles may still form in the gel during gamma sterilization, but typically disappear over a period of from about twenty-four (24) hours to about seventy-two (72) hours. Typically, mineral oil at room temperature has about ten percent (10%) dissolved gas. As discussed above, removing air from the gel makes the gel firmer. This effect is counterbalanced by a softening of the gel by the gamma radiation during gamma sterilization, however.

In some examples in which the gel pad **10530** is gamma sterilized, the gel comprises about ninety percent (90%) mineral oil by weight and about ten percent (10%) KRATON^{®} by weight. As stated above, degassing the slurry makes the gel firmer. The counteracting softening by the gamma radiation, however, results in a gel with substantially the same firmness as a gel comprising about ninety percent (90%) mineral oil by weight and about ten percent (10%) KRATON^{®} by weight that is not degassed and gamma sterilized.

In some examples, the gel pad **10530** is coupled to, attached to, formed with, or integrated with the cap ring **10510** to provide a gas-tight seal between the cap ring **10510** and the tubular body **6130** (FIG. **6A****), 7130** (FIG. **6D****).** The gel pad **10530** covers and seals the entire opening in the cap ring **10510,** as well as covering substantially the entire wound or orifice opening. As stated above, the gel pad **10530** provides a gas tight seal around a variety of shapes and sizes of instruments inserted there through.

Examples in which a gel pad support structure of the cap ring **10510** comprises a thermoplastic elastomer, for example, DYNAFLEX^{®} or KRATON^{®}, and the gel pad **10530** comprises a similar thermoplastic elastomer, for example, KRATON^{®}, exhibit improved adhesion between the gel pad **10530** and the cap ring **10510.** The polystyrene component of KRATON^{®} in the gel pad **10530** improves adhesion with polyphenylene oxide (PPO), polystyrene, and other similar polymers.

In some examples of cap rings **10510** comprising polycarbonate, the polycarbonate component of the cap ring **10510** does not bond with the gel pad **10530** at 130 °C, which is a typical manufacturing temperature for a gel pad **10530** comprising KRATON^{®}. Raising the temperature to about 150 °C for a few minutes during casting, however, bonds the gel pad **10530** to the cap ring **10510.** It is believed that heating the gel pad **10530** and cap ring **10510** to a temperature at which both the polystyrene component of the gel and the polycarbonate are simultaneously above their melt points allows bonds to form there between. In other examples, the uncured gel and the cap ring **10510** are heated to near or at the glass transition temperature of the polycarbonate in the cap ring **10510,** thereby bonding the gel pad **10530** to the cap ring **10510.**

In some examples, the gel comprises mineral oil and the cap ring **10510** comprises a polymer that dissolves in mineral oil under the manufacturing conditions, for example, polyethylene (PE), low density polyethylene (LDPE), high density polyethylene (HDPE), and ultra high molecular weight polyethylene (UHMWPE). Using polyethylene (PE) as an example, PE has a higher molecular weight than mineral oil and dissolves in mineral oil at the temperatures used to cast the gel pad **10530.** As such, as a portion of the PE in the cap ring **10510** dissolves in the mineral oil in the gel pad **10530** at the processing temperatures, for example, above about 130 °C, a bond between the PE in the cap ring **10510** and gel pad **10530** is formed.

In an example of a method for manufacturing a gel cap, the cap ring **10510** is placed into a mold that together with the cap ring **10510** includes a negative space in the desired shape of the gel pad and uncured gel is added to the mold. Sufficient uncured gel is then added to the mold to cover and fill the apertures **10512.** The uncured gel flows through, fills, and remains within the apertures. Also, in some examples, the mold is filled with sufficient uncured gel to extend into the distal portion of the cap ring **10510.** After the gel cures, the gel in the apertures connects and couples the gel on a first side of each aperture **10512to** the gel on a second side of the aperture, thereby mechanically locking the gel pad **10530** to the cap ring **10510.**

Some examples include another method for coupling the gel pad **10530** to the cap ring**10510,** either in addition to or instead of the mechanical interlocking discussed above. Such methods are useful, for example, for coupling separately formed gel pads or gel slugs **10530** and cap rings **10510.** Some examples use a glue or adhesive to couple the gel pad **10530** to the cap ring **10510,** for example, cyanoacrylate (SUPERGLUE^{®} or KRAZY GLUE^{®}). The glue is believed to bond to either the rubber or the styrene component of the triblock copolymer with a bond is frequently stronger than the gel material itself. Some examples use solvent welding in which a solvent dissolves a plastic in the cap ring **10510** and the polystyrene in the gel pad **10530.** The solvent is applied to the gel pad **10530** and cap ring **10510** by any suitable method, for example, by spraying and/or by dipping. In effect, the solvent melts both the plastic of the cap ring **10510** as well as the polystyrene in the gel pad **10530,** thereby forming a bond between the two, which remains after the solvent evaporates.

In an example for manufacturing a gel cap **10500,** the gel pad **10530** is cast into the cap ring **10510** to form the gel cap **10500.** The cap ring **10510** is positioned in or placed into a mold cavity of a casting mold. Examples of the mold cavity include support for the annular walls of the cap ring **10510.** Examples of the mold comprise a material with sufficient heat dissipation properties, for example, at least one of aluminum, copper, and brass. Those skilled in the art will recognize that other mold materials with lower heat dissipation properties will produce acceptable parts in some examples. Furthermore, some examples of the mold comprise active cooling elements, for examples, channels through which coolants are pumped.

The mold cavity and cap ring **10510** assembly is then filled with a desired amount of the triblock copolymer/mineral oil slurry such that the slurry contacts the cap ring **10510.** In some examples, the slurry is preheated, for example, to about 52 °C (125 °F), which facilitates a complete filling of the mold cavity by the slurry, thereby reducing the probability of voids in the gel. Preheating the slurry to a temperature below the MGT reduces the viscosity of the slurry and allows the slurry to flow more easily. As stated above, some examples of the slurry are degassed in a vacuum before casting. In some examples, the slurry is also degassed after it is filled in the mold cavity to remove any air that may have been introduced during the filling of the mold cavity, as well as to facilitate flow of the slurry into voids in the mold. The mold, cap ring, and slurry are heated, for example, in an oven, until the slurry reaches a temperature of about 150 °C. As stated above, the slurry turns into gel at about 120 °C; however, at about 150 °C, the gel bonds to a polycarbonate cap ring **10510.** Depending on the material used in the cap ring **10510,** bonding may take place at a temperature other than about 150 °C. In examples in which the cap ring **10510** is comprises a material with a lower melting point than the MGT, for example 120 °C, the gel pad **10530** is molded separately as a gel slug, which is then bonded to the cap ring **10510** as discussed above.

When the transformation of the slurry into a gel is complete, for example, when the temperature of the gel pad reaches about 150 °C, the gel cap **10500** is cooled, for example, by air-cooling, cold-water immersion, or another suitable method. At 150 °C the gel pad **10530** is soft and easily distorted. Distortions in the gel pad **10530** present during cooling would be set after cooling. Accordingly, in some examples, the gel cap **10500** is cooled within the mold, thereby reducing the likelihood of distorting the gel pad **10530.** Factors affecting the cooling time include the size and configuration of the mold, the quantity of gel, temperature and quantity of cooling medium, the properties of the cooling medium, and the mold material. As an example, the cooling time for a particular gel cap **10500** may be about two (2) hours for air cooling and about fifteen (15) minutes for water cooling. Whether cooling with air or water, the final properties of the gel are substantially the same. The gel cap **10500** is typically cooled to about ambient room temperature, but may be cooled to a lower temperature if desired. At about 0 °C, the gel hardens, which is useful, for example, in secondary operations such as when coupling separately manufactured gel pads **10530** and cap rings **10510.** The gel cap **10500** may be removed from the mold at any time after the gel has set.

When removed from the mold, the gel pad **10530** typically has a tacky surface. Coating the gel pad **10530** with a powder, such as cornstarch, substantially reduces or eliminates the tackiness of the cured gel pad **10530.**

As stated above, in some examples, the gel pad **10530** is molded separately from the cap ring **10510,** and coupled to the cap ring **10510** in a secondary operation, for example, bonding. In some examples, the gel pad **10530** is molded as a gel slug with an outer perimeter smaller than the perimeter of the inner cylindrical wall of the cap ring **10510** and a height greater than the height of the cap ring **10510.** Because the gel pad **10530** is molded separate from the cap ring **10510,** the slurry need only be heated to the MGT, for example, about 120 °C, to complete the transformation of the slurry into a gel, whereupon the gel becomes substantially transparent. As discussed above, the gel slug may be cooled, for example, to about 0 °C, then placed within the inner cylindrical wall of the cap ring **10510.**

In some examples, the gel slug is coupled to the cap ring **10510** through compression molding, in which the gel slug is compressed longitudinally, thereby expanding the outer perimeter of the gel slug and compressing the gel slug against the inner cylindrical wall of the cap ring **10510.** The compressed gel slug and cap ring **10510** are then heated to a sufficient temperature for the polystyrene in the gel and the polymer of the cap ring **10510** to form bonds there between. Molding the gel slug separately from the cap ring **10510** followed by heat bonding the gel slug to the cap ring is especially useful in examples in which the cap ring **10510** comprises a material with a melting temperature lower than the MGT of the gel. In such situations, the gel slug can be molded separately and heat bonded to the cap ring **10510** without melting the cap ring **10510.**

An example of a method for retracting an incision or body orifice using the retractor **6100, 7100** is discussed in detail above. Methods do not form part of the claimed invention. The method results in the outer ring **6120, 7120** of the retractor substantially in contact with the exterior surface of the body wall. The gel cap **10510** is then coupled to the outer ring **6120, 7120** of the retractor, thereby sealing the opening between the body cavity and the area outside the body cavity and allowing the surgeon to insufflate the body cavity.

As discussed above, examples of the gel cap **10500** comprise no preformed access channels in the gel pad **10530.** In use, instruments may be inserted directly through the gel pad **10530,** thereby creating access channels through the gel pad **10530.** Each access channel created in the gel cap forms an instrument seal in the presence of an instrument passing there through because the gel provides a gas tight seal around a variety of shapes and sizes of instruments. When the instrument is removed from the gel pad **10530,** the channel created in the gel pad by the instrument closes to form a zero seal.

Some examples of the cap use access devices such as trocars inserted through the gel pad **10530** for instrument access, in particular, where an access channel experiences repeated instrument manipulation, for example, insertion, removal, advancement, retraction, rotation and/or other manipulation. Each trocar inserted through the gel pad **10530** permits repeated introduction, removal, and/or manipulation of instruments there through.

In some examples, the gel cap **10500** initially comprises no access channels, and the surgeon is at liberty to determine the placement of instruments there through. Moreover, the surgeon has unlimited flexibility in the placement and repositioning of ports within the area of the gel cap **10500,** as well as the option of selecting different trocar sizes for different clinical procedures. Being detachable, the gel cap **10500** allows for the removal of large specimens. Once removed, the gel cap **10500** can be re-coupled to the outer ring **6120, 7120** of the retractor, thereby restoring the seal and allow the surgeon to reinsufflate the body cavity.

Moreover, examples of the gel are deformable without losing physical integrity, and while maintaining substantially gas tight instrument seals with any instruments extending there through, as well as gas tight zero seals for any access channels without any instruments extending there through. Accordingly, examples of the gel cap **10500** permit both translational or positional, and angular or pivotal "float" or degrees of freedom for the instruments passing through the gel pad **10530.** This float permits instrument motion both relative to the cap ring **10510** as well as relative to other instruments. In contrast, other single or limited port systems do not exhibit one or both translational or angular float for instruments.

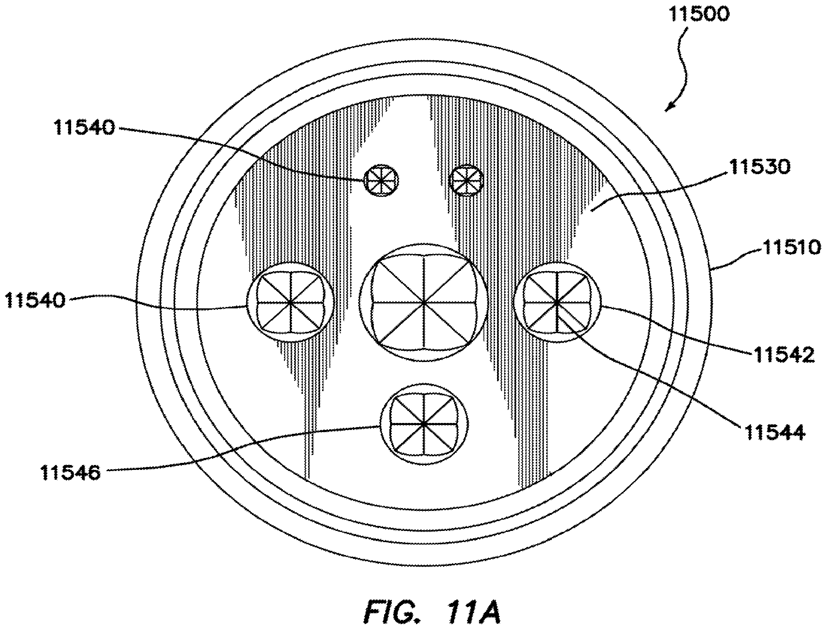

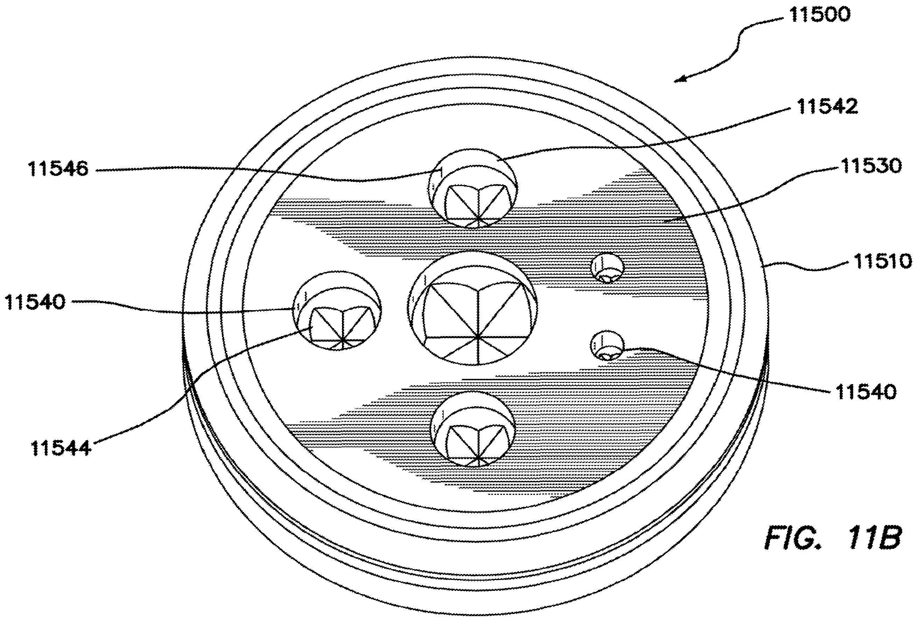

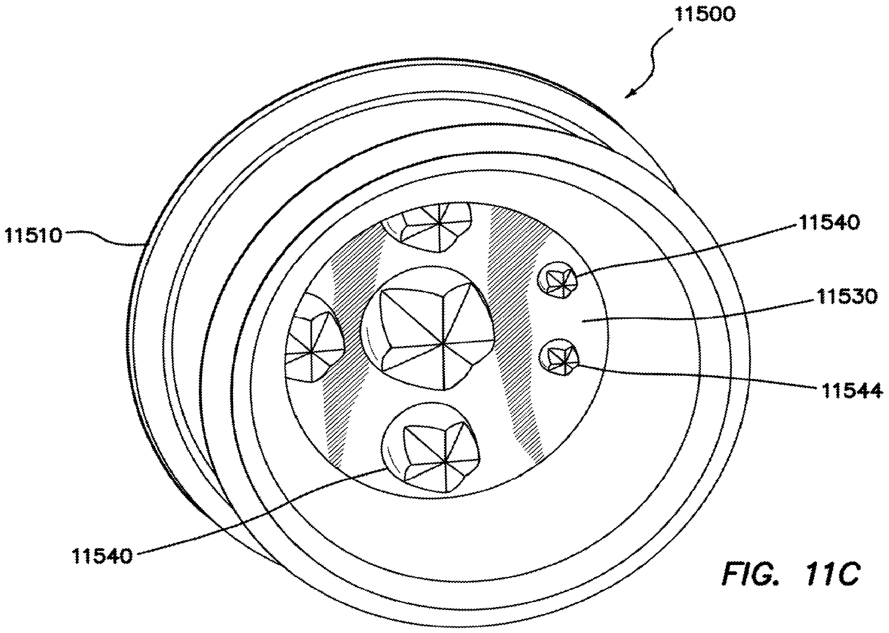

FIG. **11A** is a top view of an example of a gel cap **11500** comprising a plurality of access ports, seals, or sealing valves disposed in the gel pad. FIG. **11B** is a perspective top view of the gel cap **11500** mounted on a retractor. **FIG.11C** is a perspective bottom view of the gel cap **11500** mounted on a retractor. The gel cap **11500** comprises a cap ring **11510** and a gel pad **11530,** which are generally similar to the cap ring and gel pad of the example described above.

The gel cap **11500** further comprises a plurality of access ports **11540,** at least a portion of which is disposed within or embedded within the gel pad **11530.** In the illustrated example, the access ports **11540** have a low profile, that is, do not protrude or protrude minimally above the proximal surface of the gel pad **11530** and/or below the distal surface of the gel pad **11530.** Accordingly, the lengths of the access ports **11540** are similar to the thickness of the gel pad **11530,** which is shorter than a length of a typical trocar inserted in the gel pad **11530,** which comprises a seal assembly positioned above the gel pad **10530,** and a cannula extending through the gel pad **11530.** The reduced length of the access port **11540** allows increased angular or pivotal motion for instruments extending there through, and also permits the use of curved and/or angled instruments. In the illustrated example, the access ports **11540** are substantially permanent or non-removable under the conditions under which the gel cap **11500** is used. Trocars can also be inserted through the gel pad **11530** if additional ports are desired.

Each port **11540** comprises longitudinal axis extending from a proximal side to a distal side of the gel pad **11530,** a first seal **11542** disposed at the proximal side of the gel pad **11530,** and a second seal **11544** disposed distal to the first seal **11542.** A sight of each of the ports or seals **11540** has an aperture through the gel pad **11530** and coincides with the longitudinal axis. In the illustrated example, the first seal **11542** forms an instrument seal with an instrument extending there through and the second seal **11544** forms a zero seal in the absence of an instrument extending there through.

In the illustrated example, the first seal **11542** comprises a septum seal. Each septum seal comprises an aperture **11546** there through that is slightly smaller than a cross-section of the smallest instrument to be inserted there through. The aperture **11546** of the septum seal is substantially aligned with the aperture through the gel pad and the longitudinal axis of the port **11540.** When an instrument is inserted through the aperture **11546** of the septum seal, the aperture **11546** expands and engages the outer surface of the instrument, thereby forming a seal therewith. The septum seal comprises an elastomeric material that biases the aperture against an instrument is inserted there through. Those skilled in the art will understand that other types of instrument seals are used in other examples.

In the illustrated example, the second seal **11544** comprises a double-duckbill valve, which functions as a zero-closure seal that provides a zero seal in the absence of an instrument inserted there through. Those skilled in the art will understand that the second seal comprises another type of seal, for example, a duckbill valve, a flap valve, and the like. The double-duckbill valve comprises as elastomeric material. In some examples, each of the first seal **11542** and the second seal **11544** independently comprise an elastomeric material, for example, at least one of rubber, synthetic rubber, silicone, ethylene propylene diene monomer (EPDM), ethylene-propylene copolymer (EP rubber), polyisoprene, polybutadiene, polyurethane, styrene-butadiene, ethylene vinyl acetate (EVA), polychloroprene (NEOPRENE^{®}), perfluorelastomer (KALREZ^{®}), and the like

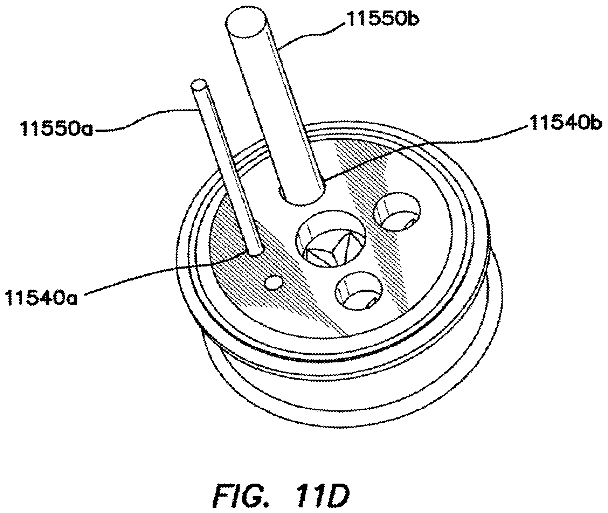

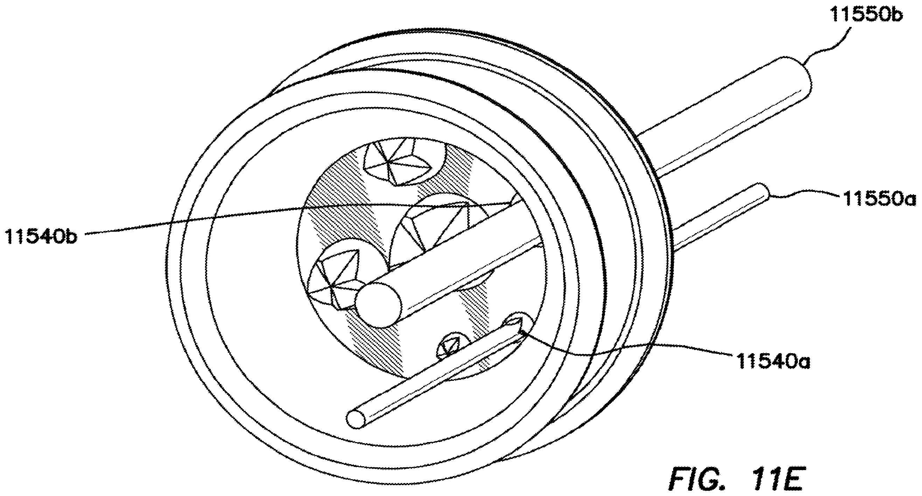

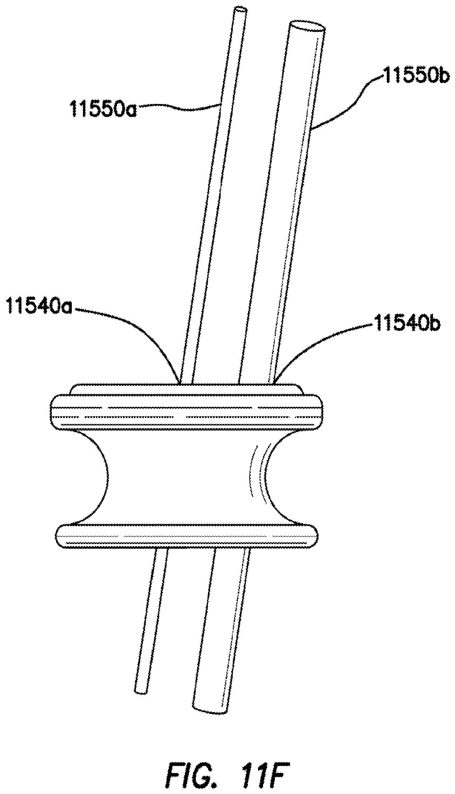

Thus, during use, the septum seal provides an instrument seal in the presence of an instrument inserted there through, and the duckbill valve provides a zero seal in the absence of an instrument inserted there through. The illustrated example comprises ports or seals **11540** in the gel pad of different sizes. Each size of port **11540** sealing accommodates a different range of instrument sizes inserted there through. The size of a port is typically given as the diameter of the largest instrument that the port will accommodate, for example, 5 mm, 11 mm, or 12 mm. FIGS. **11D****,** **11E****,** and **11F** are a perspective top view, a perspective bottom view, and a side view of a thinner instrument **11550***a* and a thicker instrument **11550***b* inserted through a smaller port **11540***a* and a larger port **11540b,** respectively, of the example of the gel cap **11500** illustrated in FIGS. **11A-11C****.**

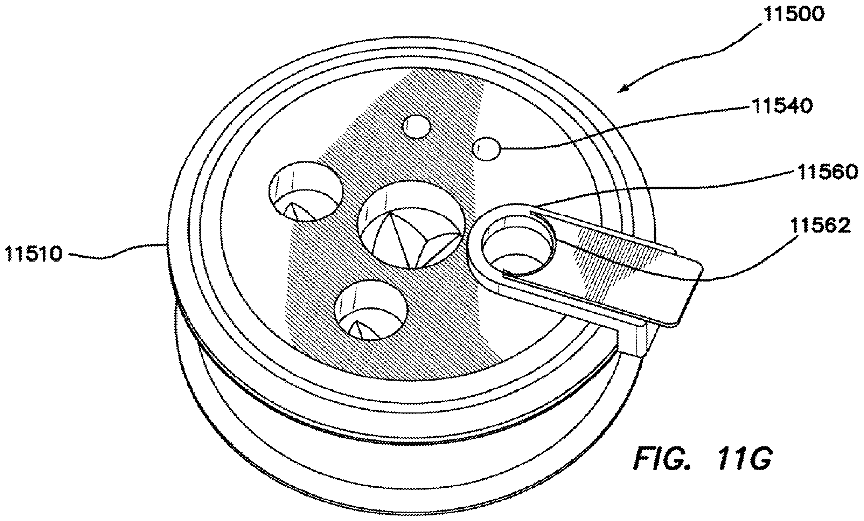

FIG. **11G** is a top perspective view of an example of a gel cap **11500** further comprising a fixed port position, for example, for a camera or a laparoscope. The fixed port **11560** comprises a lock mechanism **11562** that maintaining the position of a camera or laparoscope inserted there through. In some examples, one of the ports **11540** further comprises a stopcock and/or gas fitting used as a gas inlet and/or outlet port for insufflating, depressurizing, and/or venting the body cavity of gas. In some examples, a gas inlet/outlet port is disposed on the cap ring **11510.**

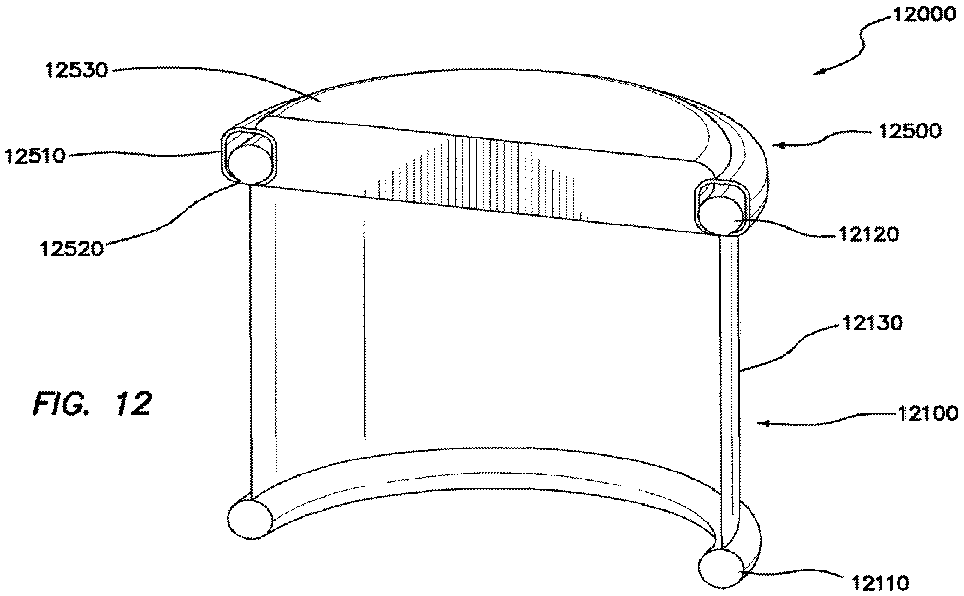

FIG. **12** is a cutaway perspective view of an example of an access device system **12000** comprising retractor **12100** and a cap or cover **12500,** which are similar to examples of retractors and gel caps described above. The retractor **12100** comprises an inner ring **12110,** an outer ring **12120,** and a sleeve **12130** extending between the inner ring **12110** and the outer ring **12120.** In the illustrated example, the cap **12500** is a gel cap comprising a proximal side, a distal side, a cap ring **12510,** and a gel pad **12530.** In the illustrated example, the cap ring **12510** comprises a tubular ring dimensioned to receive the outer ring **12120** of the retractor therewithin. The distal side of the cap ring **12510** comprises an annular slot **12520,** which is sufficiently radially deformable for the outer ring **12120** to reversibly pass there through. Accordingly, the illustrated example of the cap ring **12510** secures the cap **12500** to the outer ring **12120** with a snap or friction fit.

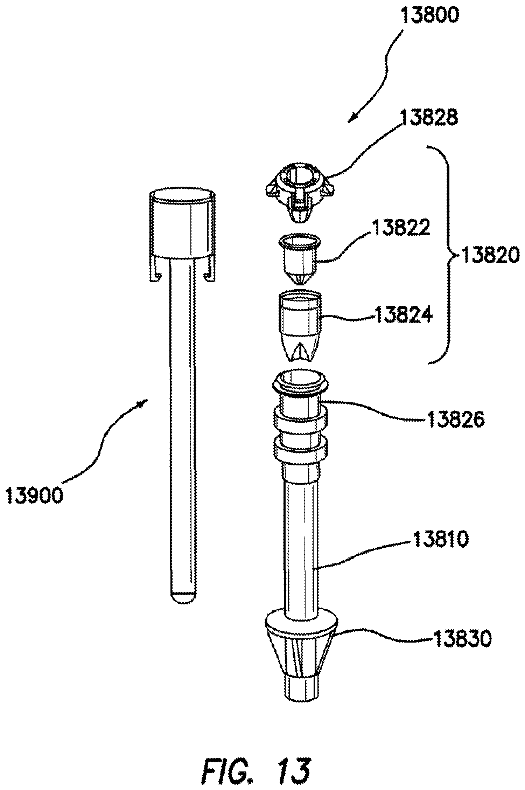

FIG. **13** is an exploded view of an example of a trocar **13800** and optional obturator **13900,** which is a component of some examples of the access device system. In the illustrated example, the obturator **13900** comprises a pointed, puncture tip **13910.** In examples in which the trocar **13800** and obturator **13900** are inserted through a gel pad **10530** rather than a body wall, potential damage to underlying tissue by contact with the tip **13910** is reduced because the gel pad **10530** serves as an artificial body wall that is spaced from the underlying tissue as discussed above. In other examples, the obturator tip **13910** has another shape, for example, blunt and/or bladeless, which, for example, reduces the likelihood of damage to other components of the access system, for example, a retraction sheath of a retractor.

The trocar **13800** comprises a proximal end, a distal end, and a longitudinal axis. The trocar **13800** comprises a cannula **13810** extending along the longitudinal axis. A trocar seal **13820** is disposed at the proximal end of the cannula **13810.** A retainer **13830** is disposed at the distal end or tip of the cannula **13810.** In the illustrated exmaple, the distal end or tip of the cannula **13810** is not angled. Other examples comprise an angled distal end or tip of the cannula **13810.** The illustrated example of the trocar **13800** does not comprise an insufflation gas inlet. Consequently, the trocar **13800** is typically used in procedures in which a body cavity is not insufflated, or in which insufflation is provided through another device..

The cannula **13810** comprises an elongate, tubular cannula body **13812** dimensioned to accommodate an instrument or instruments received there through. In the illustrated example, the cannula body **13812** is a substantially cylindrical tube, and extends through the gel pad **10530** in use. In the illustrated example, the cannula body **13812** extends from the proximal end of the cannula **13810** to which the trocar seal **13820** is coupled, and which has a larger outer diameter than the cannula body **13812.**

In some examples, the cannula **13810** is comparatively short because the cannula body **13812** need only traverse the gel pad **10530** (FIG. **10A****),** which has a known and consistent thickness, rather than a body wall. Accordingly, some examples of the cannula body **13812** are not more than about 2-times longer, about 1.5-times longer, about 1.2-times longer, or about 1.1-times longer than the thickness of the gel pad. In some examples, the cannula body **13812** is less than about 20 mm, about 10 mm, or about 5 mm longer than the thickness of the gel pad. In some examples, the cannula body **13812** is about as long as the gel pad is thick. In other examples, the cannula body **13812** has a different length, for example, a length typical for a cannula used for traversing a body wall. Shorter length cannula bodies permit increased angular degrees of freedom for instruments passing there through. Examples of shorter cannula bodies also accommodate curved instruments. The cannula **13810** comprises any suitable biocompatible material. In some examples, the cannula **13810** comprises a flexible material.

The illustrated trocar seal **13820** comprises an instrument or septum seal **13822** and a zero seal **13824.** The instrument seal **13822** seals instruments passing there through, thereby maintaining pressurization in a body cavity such as pneumoperitoneum or pneumorectum. The zero seal **13824** provides a seal when no instrument passes through the trocar seal **13820.** The instrument seal **13822** and zero seal **13824** are received in a housing **13826** disposed at the proximal end of the cannula **13810** and secured therein by a seal cover **13828.**

The retainer **13830** is disposed at or near the distal end of the cannula **13810.** In the illustrated example, the distal end of the cannula **13810** is generally perpendicular to the longitudinal axis thereof, or not angled. Other examples comprise an angled distal end or tip. In some examples, the retainer **13830** and cannula **13810** are integrated, while in other examples, the retainer **13830** and cannula **13810** are not integrated. In the illustrated example, the proximal end of the retainer **13830** comprises a flange **13832** that is generally flat and perpendicular to the longitudinal axis, while the distal end is tapered, narrowing toward the distal end of the cannula **13810.** The flange **13832** reduces the likelihood of accidental or inadvertent removal of the trocar **13800** from the gel pad. Some examples of the proximal face of the flange **13832** comprise additional anchoring features, for example, at least one of barbs, spikes, ridges, texturing, and the like, which are configured to penetrate or bite into a distal face of the gel pad **10530.** In some examples, a diameter of the flange **13832** is from about 1.5 to about 2.5 times wider, or from about 2 to about 2.2 times wider than an outer diameter of the cannula body **13812.** Some examples of the trocar **13800** are 5-mm trocars, in which the outer diameter of the cannula body **13812** is from about 7 mm to about 8 mm.

The tapered end of the retainer **13830** facilitates insertion of the trocar **13800** through the gel pad, either by itself, or when assembled with the obturator **13900** extending there through. For example, in some examples, the retainer **13830** is inserted through a preformed opening in the gel pad **10530.** Because examples of the gel material of the gel pad **10530** have high elongation values, as discussed above, the retainer **13830** is insertable through a relatively small opening in the gel pad **10530,** yet resists inadvertent removal, as discussed above.

In some examples in which the retainer **13830** and cannula **13810** are not integrated, that is, are separate components, the retainer **13830** is secured to the cannula **13810** after the cannula **13810** is inserted through the gel pad. In some examples, the cannula **13810** and retainer **13830** are secured mechanically, for example, using latches, screw threads, clips, lock rings, ratchets, and the like. In some examples, the cannula **13810** and retainer **13830** are secured adhesively. In some examples, the position of the retainer **13830** is adjustable, for example, to accommodate gel pads of different thicknesses. In some examples, the cannula **13810** and/or retainer **13830** is secured to the gel pad, for example, adhesively.

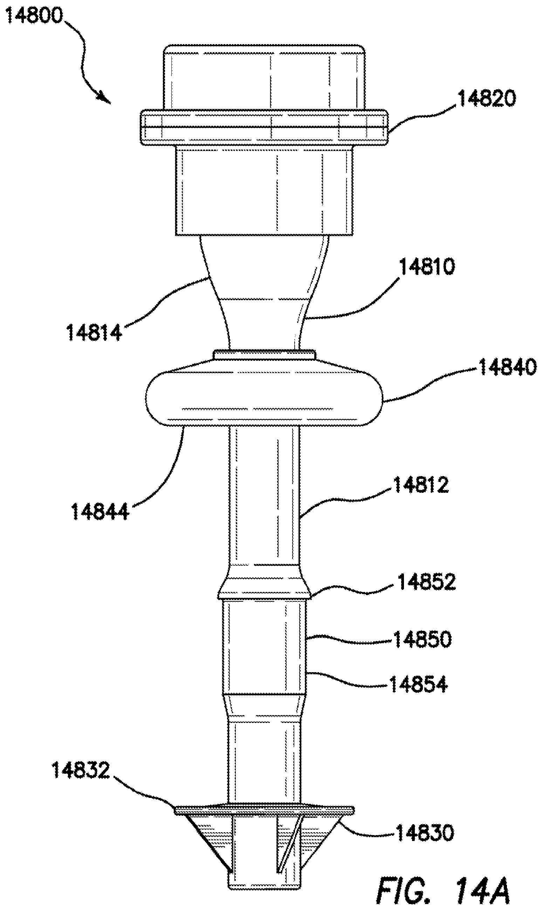

FIG. **14A** is a side view of another example of a trocar **14800** that is suitable as a component of a single-port surgical access system described above, for example, comprising a gel pad **10530** and retractor. Some examples of the access system comprise a plurality of trocars **14800.** The trocar **14800** is generally similar to the trocar **13800** described above, and comprises a cannula **14810,** a trocar seal assembly **14820,** and a retainer **14830,** which are generally similar to the corresponding features described above. The illustrated example of the trocar **14800** further comprises a bolster **14840** and a locking component **14850.** The illustrated example of the cannula **14810** is also referred to as a "fixation cannula" as will become apparent from the discussion below.

In the illustrated example, the bolster **14840** comprises a torus or doughnut. A cannula body **14812** extends through an opening in the bolster **14840.** A diameter of the opening of the bolster **14840** is sufficiently larger than an outer diameter of the cannula body **14812** to permit free movement along the cannula body **14812.** The illustrated example of the bolster **14840** comprises a deformable material, for example, a polymer resin and/or elastomer, as will be described in greater detail below. Examples of suitable materials include rubber, natural rubber, synthetic rubber, polyisoprene, styrene-butadiene rubber, silicone rubber, ethylene-propylene copolymer, ethylene-propylene-diene monomer rubber, polybutadiene, polychloroprene, polyurethane, and the like. Some examples of the bolster **14840** comprise a lubricious layer or coating in an area or region that contacts the cannula **14810,** which facilitates movement along the cannula **14810.**

An outer diameter of some examples of the bolster **14840** is from about 0.8 to about 2 times, or from about 1 to about 1.5 times a diameter of a flange **14832** of the retainer **14830.** A thickness of the bolster is from about 3 mm (0.12 inch) to about 10 mm (0.4 inch), or from about 4 mm (0.16 inch) to about 6 mm (0.24 inch). In some examples, a distal face **14844** of the bolster is concave, thereby providing additional clamping or fixation force on the gel pad **10530,** as well as conforming to gel pads **10530** with different and/or non-uniform thicknesses. The particular dimensions of the bolster **14830** are selected based on the properties of the bolster material and the gel material, and the dimensions of the cannula body **14812,** the locking component **14850,** and the gel pad **10530.**

The locking component **14850** is disposed on the cannula body **14812** proximal of the retainer **14830,** and comprises a lip **14852** proximal of an enlarged section **14854.** The lip **14852** extends radially from the cannula body **14812** with a diameter greater than the diameter of the opening of the bolster **14840.** The elastomeric material of the bolster **14840** permits the bolster **14840** to be urged over and past the lip **14852.** In the illustrated example, the lip **14852** comprises a ratchet dimensioned to facilitate the bolster **14840** sliding distally and to resist the bolster **14840** from sliding proximally. Also, in the illustrated example, the lip **14852** is a continuous structure encircling the cannula body **14812.** In other examples, the lip **14852** comprises a plurality of structures disposed around the cannula body **14812.**

The enlarged section **14854** is generally cylindrical with a diameter that is about the same as or slightly larger than the diameter of the opening in the bolster **14840,** thereby frictionally engaging the bolster **14840** thereto. In the illustrated example, the enlarged section **14854** is longer than a thickness of the bolster **14840.** In the illustrated example, the enlarged section **14854** does not extend to or contact the flange **14832** of the retainer **14830,** thereby not reducing a surface area of a proximal face thereof, and thereby improving the removal resistance thereof. In other examples, the enlarged section **14854** extends to the retainer **14830.** Other examples do not comprise an enlarged section.

A distance between a distal end of the lip **14852** and a proximal face of the flange **14832** is equal to or slightly less than a sum of a thickness of the bolster **14840** and the gel pad **10530.** In some examples, the gel pad is from about 5 mm (about 0.4 inch) to about 30 mm (about 1.2 inch) thick, or from about 13 mm (about 0.5 inch) to about 25 mm (about 1 inch) thick.

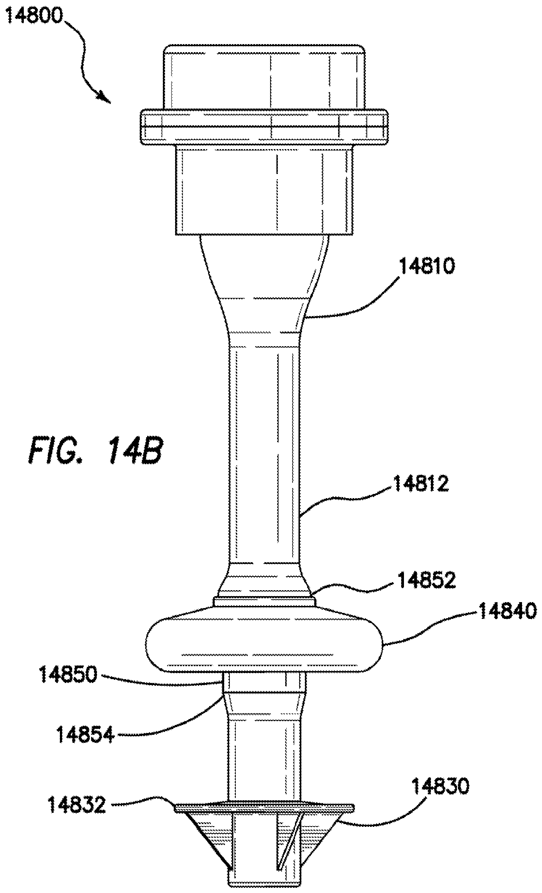

The trocar **14800** has at least two configurations: a first or insertion configuration illustrated in FIG. **14A****,** and a second or fixation configuration illustrated in FIG. **14B****.**

In an example of a method (not in accordance with the present invention) for using the trocar **14800,** the trocar **14800** is placed in the insertion configuration in which the bolster **14840** is first positioned on the cannula body **14812.** The trocar **14800** is placed in the artificial body wall either before the artificial body wall is coupled to a patient's body and/or after coupling thereto.

In the example illustrated in FIG. **14A****,** the bolster **14840** is positioned at the proximal end of the cannula body **14812,** where the bolster **14840** frictionally engages a distal portion of a cannula bell **14814,** which is an enlarged portion at the proximal end of the cannula **14810** to which the seal assembly **14820** couples.

The distal end of the trocar **14800** is positioned on, then the retainer **14830** inserted through an artificial body wall, for example, a gel pad **10530.** In some examples, an obturator **13900** (FIG. **13****)** is first inserted through the seal assembly **14820** at the proximal end of the trocar with the tip **13910** extending from the distal end thereof before this step. In other examples, an opening is first made in the artificial body wall using another instrument. In other examples, the distal end of the trocar **14800** is forced through the artificial body wall, generating an opening in the process.

The trocar **14800** is then converted into the fixation configuration illustrated in FIG. **14B** by sliding the bolster **14840** down the cannula body **14812,** and over the lip **14852** onto the enlarged section **14852.** In the illustrated configuration, the artificial body wall is captured and compressed between the flange **14830** of the retainer and the bolster **14840.** The lip **14852** locks the bolster **14840** in place, preventing it from moving proximally, thereby fixing or locking the trocar **14800** to the artificial body wall.

In the fixation configuration, the trocar **14800** fixed relative to a local portion of the artificial body wall to which it is engaged. As discussed above, however, examples of artificial body walls exhibit high elongations. Accordingly, the trocar **14800** is translatable and/or pivotable relative to an original position and orientation by deforming the artificial body wall.

In examples using an obturator **13910,** the obturator is withdrawn. The trocar **14800** serves as an access port for one or more instruments during a surgical procedure.

If desired, the trocar **14800** is removed from the artificial body wall, for example, by first disengaging the bolster **14840** from the locking component **14850,** then pulling the retainer **14830** from the artificial body wall. In some examples, the trocar **14800** and artificial body wall are not disengaged and are disposed of as a unit. In some examples, the bolster **14840** is not disengagable from the locking component **14850.**

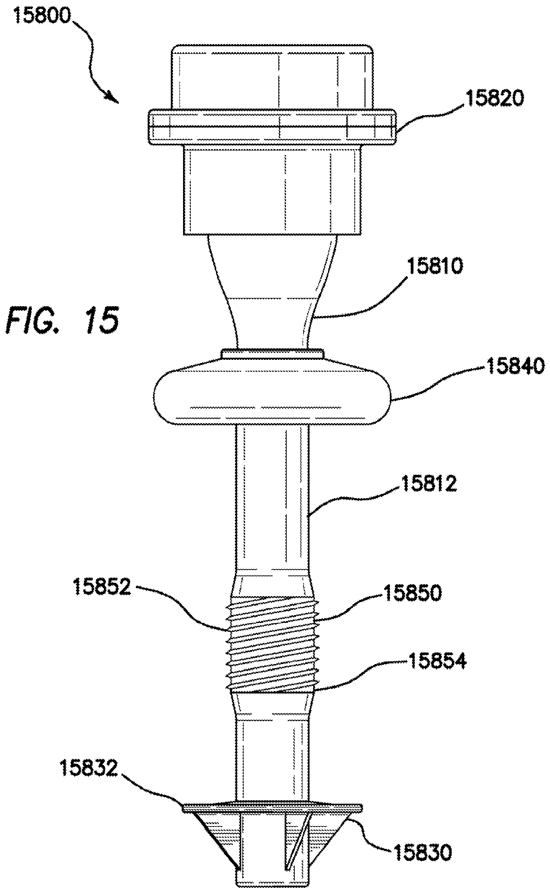

FIG. **15** is a side view of another example of a retention trocar **15000,** which is generally similar to the example illustrated in FIGS. **14A** and **14B** and described above. The trocar **15000** comprises an elongate, tubular cannula **15810** comprising a proximal end, a distal end, and a cannula body **15812;** a seal assembly **15820** coupled to the proximal end of the cannula **15810;** a retainer **15830** disposed at the distal end of the cannula **15810;** a bolster **14840** through which the cannula body **15812** extends; and a locking component **15850** disposed on the cannula body proximal of the retainer **15830.**

In the illustrated example, the locking component **15850** comprises an enlarged section **15854** on which are disposed screw threads **15852.** The bolster **15840** comprises matching threads. Consequently, the bolster **15840** is threadably engagable to the locking component **15850.** The threading also permits adjusting the relative positions of the bolster **15840** and a flange **15832** of the retainer in the fixation configuration of the trocar **15800,** thereby permitting fixation to an artificial body wall with a non-uniform thickness and/or to artificial body walls of different thicknesses.

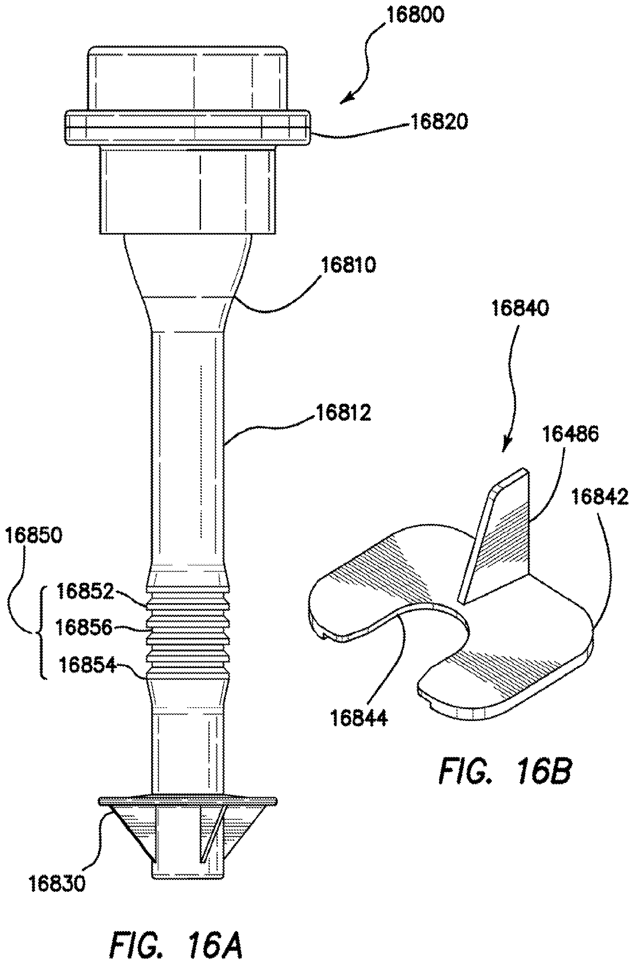

FIG. **16A** is a side view of another example of a trocar **16800.** FIGS. **16B** is a perspective view of an example of a bolster **16840** usable with the trocar **16800.** The combination of the trocar **16800** and bolster **16840** are generally similar to the examples of trocars illustrated in FIGS. **14A****,** **14B****,** and **15****.** The trocar **16800** comprises an elongate, tubular fixation cannula **16810** comprising a proximal end, a distal end, and a cannula body **16812;** a seal assembly **16820** coupled to the proximal end of the cannula **16810;** a retainer **16830** disposed at the distal end of the cannula **16810;** and a locking component **16850** disposed on the cannula body proximal of the retainer **16830.**

In the illustrated examples, the locking component **16850** comprises an enlarged section **16854** comprising a plurality of annular rings **16852** extending radially from the cannula body **16812,** which define a plurality of annular slots **16856.** In the illustrated example, a proximal edge of each ring **16856** is beveled; however, some examples do not comprise a beveled edge.

FIG. **16B** illustrates an example of a bolster **16840** in the form of a clip comprising a flattened body **16842** comprising a cut-out **16844** comprising a semicircular portion. The cut-out **16844** is dimensioned to engage the slots **16856.** A thickness of the body **16842** at the cut-out **16844** is also dimensioned to engage the slots **16856.** The bolster **16840** comprises a grip **16846** extending vertically from the body **16842,** which provides a user grip for installing and/or adjusting the bolster **16840.** In other examples, the cut-out **16844** has another shape, for example, polygonal, rectangular, a portion of a hexagon, and the like.

In use, the retainer **16830** of the trocar is inserted through an artificial body wall as discussed above, and fixed therein by engaging the bolster **16840** in a slot **16856** providing a desired fixation force. The degree of fixation is adjustable by selecting a different slot.

In some examples, the bolster cut-out **16844** engages a plurality of slots, thereby providing additional stability in the fixation configuration. Other examples comprise a bolster through with the cannula body **16812** extends, similar to the examples discussed above. In some of these examples, the locking component **16850** serves as a ratchet. The bolster comprises one or more pawls, which are optionally disengagable, thereby enhancing adjustability.

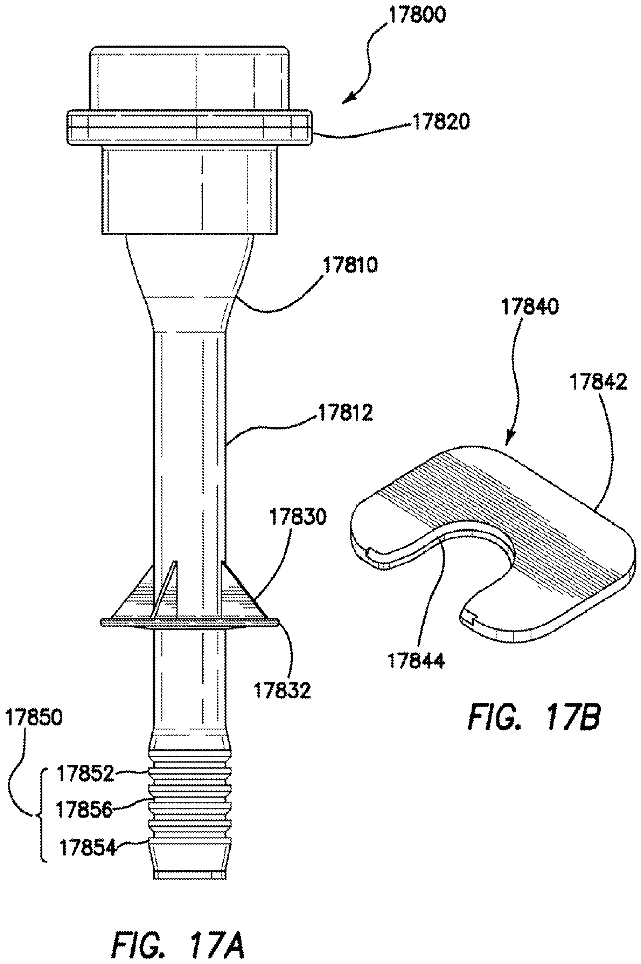

FIG. **17A** illustrates a side view of an example of a trocar **17800** comprising a fixation cannula and FIG. **17B** is a perspective view of an example of a bolster. The examples illustrated in FIGS. **17A** and **17B** are generally similar to the examples of trocars illustrated in FIGS. **14A-16B** and described above.

The trocar **17800** comprises an elongate, tubular fixation cannula **17810** comprising a proximal end, a distal end, and a cannula body **17812;** a seal assembly **17820** coupled to the proximal end of the cannula **17810;** a retainer **17830** disposed on the cannula body **17812;** and a locking component **17850** disposed at the distal end of the cannula **17810.** The illustrated example of the trocar **17800** is similar to the example illustrated in FIG. **16A** with the positions of the retainer **17830** and the locking component **17850** reversed. In the illustrated example, a flange **17832** of the retainer faces distally.

The locking component **17850** comprises an enlarged section **17854** comprising a plurality of annular rings **17852** extending radially from the cannula body **17812,** which define a plurality of annular slots **17856.**

FIG. **17B** illustrates an example of a bolster **17840** in the form of a clip comprising a flattened body **17842** comprising a cut-out **17844** comprising a semicircular portion. The cut-out **17844** is dimensioned to engage slots **17856** in the locking component. A thickness of the body **17842** at the cut-out **17844** is also dimensioned to engage the slots **17856.** The illustrated example of the bolster does not comprise a grip; however, other examples comprise a grip.

In some examples for using the example of the trocar **17800,** the cannula **17810** is fixed to an artificial body wall before the artificial body wall is coupled to a patient's body. For example, in some examples, one or more trocars **17800** are fixed on a gel pad **10530** (FIG. **10A****)** of a gel cap **10500** before the gel cap **10500** is coupled to a retractor **6100, 7100** (FIGS. **6A**-F).

While certain examples have been particularly shown and described with reference to exemplary examples thereof, it will be understood by those of ordinary skill in the art that various changes in form and details may be made therein without departing from the scope of the invention as defined by the following claims.

## Claims

1. A surgical access port system adapted for performing laparoscopic surgical procedures at a natural orifice comprising a retractor (6100) comprising:
an outer ring, wherein the outer ring is configured to be disposed proximate the natural orifice of the patient and substantially surround the orifice;
a tubular body (6130), wherein the tubular body defines a generally cylindrical passage large enough to accommodate at least one laparoscopic instrument there through;
a funnel segment (6140) extending between and coupling the outer ring and the tubular body, wherein the funnel segment (6140) provides a diametric reduction between the relatively large diameter of the outer ring and the relatively smaller diameter of the tubular body (6130), which is sized to fit within a natural orifice with minimal distention of the orifice; and
an inflatable member (6132) disposed on a distal edge of the tubular body (6130), **characterized in** further comprising:
an inflation port (6134) disposed within the funnel segment (6140); and
a channel (6136) disposed within the wall of the tubular body (6130), wherein a proximal opening of the channel communicates with the inflation port (6134) and a distal opening (6139) of the channel (6136) communicates with the inflatable member (6132),
wherein the inflatable member (6132) has a molded shape suitable for use with particular natural orifices.

2. The surgical access port system of claim 1, wherein the tubular body (6130) comprises a relatively rigid material.

3. The surgical access port system of claim 2, wherein the tubular body (6130) comprises a polycarbonate.

4. The surgical access port system of claim 1, 2 or 3, wherein the inflatable member (6132) comprises a polyolefin tubing.

5. A method for making the surgical access port system of claim 4, wherein the method comprises the steps of:
heat shrinking the polyolefin tubing around an exterior surface of the distal end of the tubular body (6130),
heating the distal end of the tubular body (6130) and placing the distal end of the tubular body (6130) inside a mold having a desired shape of the inflatable member (6132) when inflated, and
injecting a gas into the inflatable member (6132) through the inflation port (6134) and channel with the distal end of the tubular body (6130) still inside the mold to thereby produce the desired molded shape of the inflatable member (6132).

6. The surgical access port system of claim 1, wherein the inflatable member (6132) is configured to be inflated using a syringe, and wherein the syringe provides fluid or gas to the inflatable member via the inflation port (6134).

7. The surgical access port system of claim 1, wherein the inflatable member (6132), in a deflated state has a small diameter adapted for insertion and removal of a portion of the surgical access port system in the natural orifice.

8. The surgical access port system of claim 7, wherein the inflatable member (6132) further has an inflated state, and wherein the inflated state has a large diameter adapted to provide retention of an inserted portion of the surgical access port system in the natural orifice.

9. The surgical access port system of claim 1, wherein the inflation port (6134) is a normally closed check valve having a spring-loaded plunger.

10. The surgical access port system of claim 9, wherein the check valve is a Luer lock.

11. The surgical access port system of claim 1, wherein the molded shape is a toroid shape.

12. The surgical access port system of claim 1, wherein the molded shape is a disc shape.

13. The surgical access port system of claim 1, wherein the molded shape is a fluted balloon shape.

14. The surgical access port system of claim 5, wherein the molded shape is at least one of a toroid shape, a disc shape, or a fluted balloon shape based on the mold used.

## Patentansprüche

1. Operationszugangs-Port-System, das für die Durchführung von laparoskopischen chirurgischen Eingriffen an einer natürlichen Körperöffnung geeignet ist, das einen Retraktor (6100) umfasst, der Folgendes umfasst:
einen Außenring, wobei der Außenring konfiguriert ist, um nahe der natürlichen Körperöffnung des Patienten angeordnet zu werden und im Wesentlichen die Körperöffnung zu umschließen;
einen rohrförmigen Körper (6130), wobei der rohrförmige Körper einen allgemein zylindrischen Durchgang definiert, der zur Unterbringung von mindestens einem laparoskopischen Instrument dort hindurch groß genug ist;
einen Trichterabschnitt (6140), der sich zwischen dem Außenring und dem rohrförmigen Körper erstreckt und diese verbindet, wobei der Trichterabschnitt (6140) eine Durchmesserreduzierung zwischen dem relativ großen Durchmesser des Außenrings und dem relativ kleineren Durchmesser des rohrförmigen Körpers (6130) bereitstellt, der zur Einpassung innerhalb einer natürlichen Körperöffnung mit minimaler Dehnung der Körperöffnung dimensioniert ist; und
ein aufblasbares Element (6132), das an einem distalen Rand des rohrförmigen Körpers (6130) angeordnet ist,
weiter **dadurch gekennzeichnet, dass** es Folgendes umfasst:
einen Aufblasanschluss (6134), der innerhalb des Trichterabschnitts (6140) angeordnet ist; und
einen Kanal (6136), der innerhalb der Wand des rohrförmigen Körpers (6130) angeordnet ist, wobei eine proximale Öffnung des Kanals mit dem Aufblasanschluss (6134) verbunden ist und eine distale Öffnung (6139) des Kanals (6136) mit dem aufblasbaren Element (6132) verbunden ist,
wobei das aufblasbare Element (6132) eine geformte Form aufweist, die für die Verwendung mit bestimmten natürlichen Körperöffnungen geeignet ist.

2. Operationszugangs-Port-System nach Anspruch 1, wobei der rohrförmige Körper (6130) ein relativ starres Material umfasst.

3. Operationszugangs-Port-System nach Anspruch 2, wobei der rohrförmige Körper (6130) ein Polycarbonat umfasst.

4. Operationszugangs-Port-System nach Anspruch 1, 2 oder 3, wobei das aufblasbare Element (6132) einen Polyolefin-Schlauch umfasst.

5. Verfahren für die Herstellung des Operationszugangs-Port-Systems nach Anspruch 4, wobei das Verfahren die folgenden Schritte umfasst:
Warmschrumpfen des Polyolefin-Schlauchs um eine Außenoberfläche des distalen Endes des rohrförmigen Körpers (6130),
Erwärmen des distalen Endes des rohrförmigen Körpers (6130) und Platzieren des distalen Endes des rohrförmigen Körpers (6130) innerhalb eines Formteils, das eine gewünschte Form des aufblasbaren Elements (6132), wenn aufgeblasen, aufweist, und
Injizieren eines Gases in das aufblasbare Element (6132) durch den Aufblasanschluss (6134) und Kanal mit dem distalen Ende des rohrförmigen Körpers (6130) noch im Inneren des Formteils, um dadurch die gewünschte geformte Form des aufblasbaren Elements (6132) herzustellen.

6. Operationszugangs-Port-System nach Anspruch 1, wobei das aufblasbare Element (6132) konfiguriert ist, um unter Verwendung einer Spritze aufgeblasen zu werden, und wobei die Spritze Flüssigkeit oder Gas dem aufblasbaren Element über den Aufblasanschluss (6134) bereitstellt.

7. Operationszugangs-Port-System nach Anspruch 1, wobei das aufblasbare Element (6132) in einem entleerten Zustand einen kleinen Durchmesser aufweist, der für die Einführung eines Teils des Operationszugangs-Port-Systems in die natürliche Körperöffnung und dessen Entfernung daraus geeignet ist.

8. Operationszugangs-Port-System nach Anspruch 7, wobei das aufblasbare Element (6132) weiter einen aufgeblasenen Zustand aufweist und wobei der aufgeblasene Zustand einen großen Durchmesser aufweist, der für die Bereitstellung von Zurückhaltung eines eingeführten Teils des Operationszugangs-Port-Systems in der natürlichen Körperöffnung geeignet ist.

9. Operationszugangs-Port-System nach Anspruch 1, wobei der Aufblasanschluss (6134) ein normal geschlossenes Rückschlagventil ist, das einen federbelasteten Kolben aufweist.

10. Operationszugangs-Port-System nach Anspruch 9, wobei das Rückschlagventil ein Luer-Lock ist.

11. Operationszugangs-Port-System nach Anspruch 1, wobei die geformte Form eine Toroidform ist.

12. Operationszugangs-Port-System nach Anspruch 1, wobei die geformte Form eine Scheibenform ist.

13. Operationszugangs-Port-System nach Anspruch 1, wobei die geformte Form eine gerillte Ballonform ist.

14. Operationszugangs-Port-System nach Anspruch 5, wobei die geformte Form mindestens eine von einer Toroidform, einer Scheibenform oder einer gerillten Ballonform, basierend auf dem verwendeten Formteil, ist.

## Revendications

1. Système de port d'accès chirurgical adapté pour effectuer des procédures chirurgicales laparoscopiques à un orifice naturel comprenant un rétracteur (6100) comprenant :
un anneau externe, dans lequel l'anneau externe est configuré pour être disposé à proximité de l'orifice naturel du patient et entoure sensiblement l'orifice ;
un corps tubulaire (6130), dans lequel le corps tubulaire définit un passage généralement cylindrique suffisamment grand pour recevoir au moins un instrument laparoscopique à travers celui-ci ;
un segment en entonnoir (6140) s'étendant entre et couplant l'anneau externe et le corps tubulaire, dans lequel le segment en entonnoir (6140) fournit une réduction de diamètre entre le diamètre relativement grand de l'anneau externe et le diamètre relativement petit du corps tubulaire (6130), qui est dimensionné pour s'ajuster dans un orifice naturel avec distension minimale de l'orifice ; et
un membre gonflable (6132) disposé sur un bord distal du corps tubulaire (6130), caractérisé en comprenant en outre :
un port de gonflage (6134) disposé dans le segment en entonnoir (6140) ; et
un canal (6136) disposé dans la paroi du corps tubulaire (6130), dans lequel une ouverture proximale du canal communique avec le port de gonflage (6134) et une ouverture distale (6139) du canal (6136) communique avec le membre gonflable (6132),
dans lequel le membre gonflable (6132) a une forme moulée convenant à une utilisation avec des orifices naturels particuliers.

2. Système de port d'accès chirurgical selon la revendication 1, dans lequel le corps tubulaire (6130) comprend un matériau relativement rigide.

3. Système de port d'accès chirurgical selon la revendication 2, dans lequel le corps tubulaire (6130) comprend un polycarbonate.

4. Système de port d'accès chirurgical selon la revendication 1, 2 ou 3, dans lequel le membre gonflable (6132) comprend du tube en polyoléfine.

5. Procédé de fabrication du système de port d'accès chirurgical selon la revendication 4, où le procédé comprend les étapes consistant à :
rétrécir thermiquement le tube en polyoléfine autour d'une surface extérieure de l'extrémité distale du corps tubulaire (6130),
chauffer l'extrémité distale du corps tubulaire (6130) et placer l'extrémité distale du corps tubulaire (6130) à l'intérieur d'un moule ayant une forme désirée du membre gonflable (6132) lorsque gonflé, et
injecter un gaz dans le membre gonflable (6132) à travers le port de gonflage (6134) et le canal avec l'extrémité distale du corps tubulaire (6130) toujours dans le moule pour produire ainsi la forme moulée désirée du membre gonflable (6132).

6. Système de port d'accès chirurgical selon la revendication 1, dans lequel le membre gonflable (6132) est configuré pour être gonflé en utilisant une seringue, et dans lequel la seringue fournit un fluide ou un gaz au membre gonflable via le port de gonflage (6134).

7. Système de port d'accès chirurgical selon la revendication 1, dans lequel le membre gonflable (6132), dans un état dégonflé a un petit diamètre adapté pour introduction et retrait d'une partie du système de port d'accès chirurgical dans l'orifice naturel.

8. Système de port d'accès chirurgical selon la revendication 7, dans lequel le membre gonflable (6132) a en outre un état gonflé, et dans lequel l'état gonflé a un grand diamètre adapté pour assurer la rétention d'une partie introduite du système de port d'accès chirurgical dans l'orifice naturel.

9. Système de port d'accès chirurgical selon la revendication 1, dans lequel le port de gonflage (6134) est une soupape de retenue normalement fermée ayant un poussoir à ressort.

10. Système de port d'accès chirurgical selon la revendication 9, dans lequel la soupape de retenue est un Luer-Lock.

11. Système de port d'accès chirurgical selon la revendication 1, dans lequel la forme moulée est une forme toroïdale.

12. Système de port d'accès chirurgical selon la revendication 1, dans lequel la forme moulée est une forme en disque.

13. Système de port d'accès chirurgical selon la revendication 1, dans lequel la forme moulée est une forme de ballon cannelé.

14. Système de port d'accès chirurgical selon la revendication 5, dans lequel la forme moulée en est au moins l'une d'entre une forme toroïdale, une forme en disque ou une forme de ballon cannelé sur la base du moule utilisé.
